# EUROPEAN PATENT APPLICATION

(11) **EP 1 535 585 A2**
(43) Date of publication of application: **01.06.2005**
(21) Application number: 04104312.6
(22) Date of filing: 07.09.2004
(51) Int. Cl.: A61B 19/00, H04N 13/00

(54) **Digital camera for medical service, photography operating device and photographic image diagnosis system for medical service**

(30) Priority: 17.09.2003 JP 2003324558
(71) Applicant: Konica Minolta Medical & Graphic Inc., Tokyo 163-0512 (JP)
(72) Inventor: Tamakoshi, Yasuaki Konica Minolta Med.&Graphic Inc, Tokio, Tokyo 192-8505 (JP)
(74) Representative: Gille Hrabal Struck Neidlein Prop Roos

(57) **Abstract**

A head-mountable digital camera for medical service, provided with a camera section to photograph an object and output digital image of the object; a display section to form a virtual image of photography relevant information with regard to the photographing by the camera section on a pupil of a photographer through an optical system; and a mounting member to mount the camera section and the display section on the head of the photographer so as to introduce light of the photography relevant information indicated on the display section to the pupil together with light from the outside so that the virtual image of photography relevant information and the image of the outside are superimposed and formed on the retina of pupil of the photographer.

## Description

### BACKGROUND OF THE INVENTION

The present invention relates to a digital camera especially for medical service which is used at the time of diagnosis in the medical organization, and when an image photographed by the digital camera for medical service is stored corresponding to an electronic medical chart, to a photographic image diagnosis system for supporting the diagnosis of a doctor.

### [Background Art]

In medical facilities, normally, in a stage of doctor's question, a case where an affected part of a patient is photographed by a photographic device such as the digital camera and recorded, is not conducted. Therefore, the patient is diagnosed by depending only on a situation of the past affected part by the memory of a doctor and the information written in the medical chart.

Normally, it is necessary that the doctor's question is completed in several minutes per one patient. In contrast to that, in conventional the photographic device such as the digital camera, it is necessary that, for the photographing, after the diagnosis of the patient is stopped once, the photographing is conducted in such a manner that the camera is held by a single hand or both hands, a composition is confirmed by a view finder, and a release switch is pressed. Therefore, it is very troublesome to conduct such a photographic operation in the diagnosis, and because a flow of operation is interrupted and effective examination is obstructed, it is not photographed by the photographic device such as a digital camera, and recorded.

Further, as the digital camera, a device which has an automatic focus function, and by which the photographed image is displayed on a glass-type image display device, is known (for example, refer to Patent Document 1).

Further, recently, in the image display device, many researches and developments are conducted, and a glass-type image display device provided with a so-called see-through function by which both of an image light of the display unit by which an inputted image is displayed, and the outside light can be introduced into pupils, is proposed.

For example, there is an image display device by which an image light displayed on a reflection type liquid crystal display (Liquid Crystal Display, hereinafter, abbreviated as LCD) is reflected on a polarizing beam splitter (PBS) film, and reflected light is presented to the use's pupils through the eye-piece optical system composed of a concave lens and a convex lens (for example, Patent Document 2).

Further, there is a retina direct image-drawing device provided with a hologram formed on a lens, a light source arranged in the vicinity of the lens, a liquid crystal display arranged in the vicinity of the lens by which the image information is supplied to the hologram by irradiating the light beam from the light source (for example, refer to Patent Document 3, Patent Document 4).

Furthermore, there is a small sized and light-weight glass-type display device in which a hologram element is provided in a plate-like prism as an image display device, and the prism is commonly used as the eye-piece optical system, and the light from the display unit is made incident on the prism from the end face, and is guided to the hologram element being reflected inside of the prism, (for example, refer to Patent Document 5, Patent Document 6, Patent Document 7).

Further, it is well-known that the digital camera is provided in the center of the glasses, and an photographed image is displayed on another terminal different from the glasses (for example, refer to Patent Document 8).

Further, the body surface such as a skin and the mucosa is ordinary a translucent body, a viewing condition is differed by an illumination condition. Therefore, depending on the case, like that the observation direction is changed, or the aspect of the patient is changed, or the lighting direction, is changed, by the observation under various illumination conditions, the doctor observes the diseased part of the patient and inspects it. However, there is no method based on this by which a sufficiently adequate photographing and image storing can be conducted.

Further, it is well-known that the image in which the patient is photographed by a digital camera is made correspondent to the electronic medical chart of the patient (for example, Patent Document 9). However, it is only correspondent to the entire electronic medical chart, but because it is not correspondent to for each diagnosis recording information of each time, the judgement for which time diagnosis the photographed image is used, is difficult at once, and there is a problem that the diagnostic efficiency as a total is ill-influenced.
(Patent Document 1) Tokkaihei 11-341399.
(Patent Document 2) Tokkai 2001-305475.
(Patent Document 3) Tokkaihei 11-64781.
(Patent Document 4) Tokkaihei 11-64782.
(Patent Document 5) Tokkai 2001-264681.
(Patent Document 6) Tokkai 2001-305476.
(Patent Document 7) Tokkai 2003-161820.
(Patent Document 8) Tokkaihei 11-252419.
(Patent Document 9) Tokkai 2001-258044.

### (Disclosure of the Invention)

### (Problems that the Invention is to Solve)

However, in the above Patent Document 1, there is not the description as the diagnosis or medical service, when, for example, it is used in the medical site, the doctor can not directly examine a patient under the situation that the glass-type display device is installed, and the doctor can examine a patient only through the photographic device. A range in which the doctor can look is limited to a photographing range (image angle) of the photographic device, and a display range of the display device, and it is influenced by a performance of an instrument such as a latitude or the resolving power of the photographic device. Therefore, for example, like that a shadow in the dark portion or delicate texture can not be judged, there is a possibility that it becomes a cause of an erroneous diagnosis or a medial accident. Further, according to a display device in Patent Document 1, because eyes of the doctor is perfectly covered when it is installed for the purpose that an image photographed by the photographic device is displayed on the display device, an expression of the doctor can be read from a patient side, there is a possibility that a psychological feeling of incompatibility such as uneasiness is given, and the reliable relationship between the doctor and a patient is not built, and there is a possibility that an effective remedy is obstructed.

In Patent Documents 2 - 7, there is no description as for the diagnosis or medical service, further, there is no description of the photographic device such as a camera.

In Patent Document 8, there is no description as for the diagnosis or medical service, further, there is no description how a photographing range information, and focus information when the photographing is conducted by a camera, are displayed.

### SUMMARY OF THE INVENTION

In view of the foregoing problems, the first invention's object is to provide a digital camera for medical service by which, at the time of the diagnosis or remedy, a burden of the doctor is lightened when the diseased part of a patient is photographed, and the effective operation can be conducted.

The second invention's object is to provide a photography operating device by which, while illuminating a target range which is appropriate for the photographing by the above-described digital camera for medical service, the photographic operation can be conducted.

The third invention's object is to provide a system by which the progress of the condition of the disease, or recovery condition is effectively and easily grasped by using the recorded medical image when the medical image photographed by the digital camera is adequately and effectively stored, and a support for the diagnosis is conducted.

The above objects can be attained by the following structure of the digital camera for medical use.

The invention written in item 1 is characterized in that: the digital camera (head-mountable digital camera) for medical service is provided with; a camera section by which an image is photographed and the digital image is outputted; and a display section by which a virtual image of the photography relevant information with regard to photographing by the camera section is formed on the retina of pupils of the photographer through an optical system, and mounting means to mount the camera section and the display section in such a way that the display section forms the virtual image of the photography relevant information on the retina of pupils of the photographer through an optical system; and it is provided in such a manner that the light of the photography relevant information displayed on the display section is guided together with the light from the outside, and the virtual image of the photography relevant information is superposed on the image of the outside.

### BRIEF DESCRIPTION OF THE DRAWINGS

Fig. 1 is an upper surface view of a digital camera for medical service.
Fig. 2 is a front surface view of a digital camera for medical service.
Fig. 3 is a micro camera unit function block diagram.
Fig. 4 is a display section sectional view.
Fig. 5 is a comparison explanatory view of a geometric regular reflection to a diffraction reflection by the hologram.
Fig. 6 is a comparison explanatory view of a geometric regular reflection to a diffraction reflection by the hologram.
Fig. 7 is a longitudinal sectional view typically showing a structure of an information display optical system.
Fig. 8 is a view showing an arrangement relationship of a hologram element and a camera section.
Fig. 9 is a view showing a display area of a hologram element.
Fig. 10 is a structural view of the digital camera for medical service.
Fig. 11 is a view showing a diagnosis condition.
Fig. 12 a schematic view of a photographic image diagnosis system for medical service.
Fig. 13 is a table correlation view.
Fig. 14 is a flow chart showing operations of the photographic image diagnosis system for medical service.
Fig. 15 is a view showing an electronic patient' chart display image plane.
Fig. 16 is a schematic view of the photographic image diagnosis system for medical service in a modified example.
Fig. 17 is a schematic sectional view of a pen-light type photography operating device.
Fig. 18 is a schematic upper surface view of a pen-light type photography operating device.
Fig. 19 is a view showing an example of the display displayed on the display section when the pen-light type photography operating device is used.
Fig. 20 is a view showing an example of the appearance viewed from the photographer when the pen-light type photography operating device is used.
Fig. 21 is a view showing an electronic stethoscope.
Fig. 22 is a view showing an example of the display displayed on the display section when the electronic stethoscope is used.
Fig. 23 is a view showing an example of the appearance viewed from the photographer when the electronic stethoscope is used.

### DETAILED DESCRIPTION OF THE PREFERRED EMBODIMENT

Firstly, preferred structures to attain the above objects will be explained.

In the digital camera for medical service written in item 1, the invention written in item 2 is characterized in that: the photography relevant information includes photographing range information to indicate a range photographed by the camera.

In the digital camera for medical service written in item 2, the invention written in item 3 is characterized in that: in the camera section, the focal length can be changed; and the photographing range information corresponds to a photographing range by the focal length of the camera section at the time of photographing.

In the digital camera for medical service written in item 2 or 3, the invention written in item 4 is characterized in that: the camera section has an automatic focusing function, and is positioned in a position different from the display section; and the photographing range information corresponds to a focusing distance of the camera section.

In the digital camera for medical service written in any one of item 1 to item 4, the invention written in item 5 is characterized in that: the camera section has an automatic focusing function; and the photography relevant information includes focusing position information corresponding to a focusing position of the camera section.

In the digital camera for medical service written in one of items 1 to 5, the invention written in item 6 is characterized in that: the camera section is positioned in a position different from the display section, and the focusing position information corresponds to the focusing distance of the camera section.

In the digital camera for medical service written in any one of items 3 to 6, the invention written in item 7 is characterized in that: the camera section has an automatic focusing function; when the camera section is focused, the photography relevant information includes focusing information showing that the camera section is focused.

In the digital camera for medical service written in any one of items 3 to 6, the invention written in item 8 is characterized in that the photography relevant information includes photographing action information corresponding to a photographing action of the camera section.

In the digital camera for medical service written in any one of items 1 to 8, the invention written in item 9 is characterized in that: the digital camera comprises a photographing operation receiving means for receiving photography start instruction information from a photography operating device; the camera section conducts photographing based on the photography start instruction information received by the photographing operation receiving means, and the photography relevant information includes photography start information corresponding the receiving of the photography start instruction information.

In the digital camera for medical service written in item 9, the invention written in item 10 is characterized in that: the photography operating device sends photography preparation instruction information before sending the photography start instruction information, the photographing operation receiving means received the photography preparation instruction information from the photography operating device, and the photography relevant information includes photography preparation information corresponding to the receiving of the photography preparation instruction information.

In the digital camera for medical service written in item 9 or 10, the invention written in item 11 is characterized in that: the photography operating device sends strobe selecting information, the photographing operation receiving means receives the strobe photography information from the photography operating device and the photography relevant information includes strobe information corresponding to the received strobe photography information.

In the digital camera for medical service written in any one of items 1 to 11, the invention written in item 12 is characterized in that: the digital camera comprises a specific position information obtaining means for obtaining information regarding a specific position with regard to a photography by the camera section, and the photography relevant information includes specific position information indicating the specific position based on the information obtained by the specific position information obtaining means.

In the digital camera for medical service written in item 12, the invention written in item 13 is characterized in that: the specific position is the position of a specific marker, the digital camera comprises a position detecting means for detecting information regarding the position of the specific marker, and the photography relevant information includes marker position information indicating the information regarding the position of the marker detected by the position detecting means.

In the digital camera for medical service written in item 13, the invention written in item 14 is characterized in that: the marker is a position marker provided in correspondence to the position of a microphone of a stethoscope or a microphone.

In the digital camera for medical service written in item 15, the invention written in item 14 is characterized in that: the marker is a position marker provided in correspondence to the position of a strobe of a separated body from the digital camera for medical service or the strobe.

In the digital camera for medical service written in item 16, the invention written in item 15 is characterized in that: the digital camera comprises a strobe light irradiating direction obtaining means for obtaining information specifying irradiating direction of the strobe, and the photography relevant information includes strobe light irradiating direction information based on the information obtained by the strobe light irradiating direction obtaining means.

In the digital camera for medical service written in any one of items 1 to 16, the invention written in item 17 is characterized in that: the mounting means is a glass-type one provided with; a frame supporting the display section; a nose pad contact with the nose; and an applied part which is attached to near both ends of the frame in the left and right directions, and includes one pair of temples contacting with the ears, side head portion or back head portion.

In the digital camera for medical service written in item 17, the invention written in item 18 is characterized in that: the camera section is provided so as to position in almost center of the applied part.

In the digital camera for medical service written in item 18, the invention written in item 19 is characterized in that: the camera section is provided at a position of almost the same height as the height of pupils of the photographer.

In the digital camera for medical service written in item 18 or 19, the invention written in item 20 is characterized in that: the nose pad can be adjusted upwardly and downwardly.

In the digital camera for medical service written in items 1 to 20, the invention written in item 21 is characterized in that: the display section is provided with; a display unit which displays the information; a casing holding the display unit; and an optical system by which the light from the display unit is guided to the pupils and the virtual image of the image is provided.

In the digital camera for medical service written in item 21, the invention written in item 22 is characterized in that: the optical system is provided with a prism having at least two reflecting surfaces which are arranged in opposite to each other, and a hologram surface composed of the reflection type hologram; and at least one surface in the two reflecting surfaces is a light beam selection surface by which the transmission and reflection are selectively conducted by an incident angle, and the image light from the display unit incident on the prism is reflected between the reflecting surfaces, next, diffractively reflected on the hologram surface, and after it passes the light beam selection surface, it is guided to the pupils of the photographer.

In the digital camera for medical service written in item 22, the invention written in item 23 is characterized in that: the hologram is a volume-type and the phase-type.

In the digital camera for medical service written in item 22 or 23, the invention written in item 24 is characterized in that: the hologram has an optical power by which the image light is enlargedly projected onto the pupils of the photographer.

In the digital camera for medical service written in any one of item 22 to 24, the invention written in item 25 is characterized in that: the hologram has a diffractive reflection angle larger than the positive reflection angle on the hologram surface.

In the digital camera for medical service written in any one of items 22 to 25, the invention written in item 26 is characterized in that: the reflecting surfaces arranged in opposite to each other form an opened angle to an incident side on the prism of the image light.

In the digital camera for medical service written in any one of items 22 to 25, the invention written in item 27 is characterized in that: it is provided with a polarization correction member which corrects the polarization of the outside light transmitting the prism.

In the digital camera for medical service written in item 27, the invention written in item 28 is characterized in that: the polarization correction member is installed in the prism on the hologram surface, and has respective surfaces on the same surface as the reflecting surfaces arranged in opposite to each other.

In the digital camera for medical service written in item 27 or 28, the invention written in item 29 is characterized in that: the reflecting surfaces arranged in opposite to each other form almost parallel to each other.

In the digital camera for medical service written in any one of items 27 to 29, the invention written in item 30 is characterized in that: the reflection between the reflecting surfaces arranged in opposite to each other is the total reflection.

In the digital camera for medical service written in any one of items 22 to 30, the invention written in item 31 is characterized in that: the hologram surface is a flat surface.

In the digital camera for medical service written in any one of items 22 to 31, the invention written in item 32 is characterized in that: at least one surface of the reflecting surfaces arranged in opposite to each other is a curved surface.

In the digital camera for medical service written in any one of items 22 to 32, the invention written in item 33 is characterized in that: the diameter of a photographing lens of the camera section is smaller that the width of the display section.

The invention written in item 34 is characterized in that: a photography operating device comprises:
an operating section for a operator;
an irradiating means for irradiating in response to an operation with the operating section;
a transmitting section to transmit information to a digital camera; and
a control means to control such that the transmitting section transmits photography start instructing information in accordance with an operation with the operating section.

In the photography operating device written in item 34, the invention written in item 35 is characterized in that:
the control means controls such that the transmitting section transmits photography preparation instructing information in accordance with an operation with the operating section before transmitting the photography start instructing information.

In the photography operating device written in item 35, the invention written in item 36 is characterized in that:
the control means controls such that the transmitting section transmits instructing information of a focal length of the digital camera in accordance with an operation with the operating section.

In the photography operating device written in any one of items 34 to 36, the invention written in item 37 is characterized in that: the irradiating means includes a strobe light irradiating means for irradiating strobe light in accordance with a control by the control means, and the control means controls the strobe light irradiating means in correspondence to the control for the transmitting means to transmit the photography start instructing information in accordance with an operation with the operating section.

In the photography operating device written in item 37, the invention written in item 38 is characterized in that:
the irradiating means includes a visual diagnosis-usable irradiating means for irradiating for visual diagnosis, and
an irradiating direction of the irradiating means is almost equal to that of the visual diagnosis-usable irradiating means.

In the photography operating device written in any one of items 34 to 38, the invention written in item 39 is characterized in that: the visual diagnosis-usable irradiating means is a pen-shaped light type.

The invention written in item 40 is characterized in that: it is a photographic image diagnosis system for medial service having; a digital camera for medical service by which the diseased part of a patient is photographed and the photographed image data is outputted; terminal device which is connected to the digital camera for medical service and by which an electronic medical chart is displayed; and data base memory means connected to the terminal device, and in the data base memory means, a medical chart identification information for identifying the electronic medical chart is made correspondent for each electronic medical chart and registered, and in the electronic medical chart, a diagnosis recording information is included, and for each diagnosis recording information, a diagnosis recording information identification information is made correspondent to each other, and registered, and the terminal device selects the specific diagnosis order information from the diagnosis order information by an operation input, and displays the electronic medical chart corresponding to the selected diagnosis order information, and the photographed image data outputted from the digital camera for medical service, and the photographed image identification information are made correspondent to each other, and are registered in the data base memory means, and the photographed image identification information and the diagnosis recording information identifying information for identifying the diagnosis recording information corresponding to the photographing by the digital camera for medical service are made correspondent to each other, and registered in the data base memory means.

The invention written in item 41 is characterized in that: in the photographic image diagnosis system written in item 40 for medical service, the terminal device registers the digital camera identification information for identifying the digital camera for medical service corresponding to the photographic image identification information, in the data base memory means.

The invention written in item 42 is characterized in that: in the photographic image diagnosis system for medical service written in item 40 or 41, the terminal device registers the photographing date and hour information showing date and hour at which the digital camera for medical service photographs corresponding to the photographic image identification information in the data base memory means.

The invention written in item 43 is characterized in that: in the photographic image diagnosis system for medical service written in any one of items 40 to 42, the terminal device is a device which selects the specific diagnosis order information from the diagnosis order information by the operation input, and has a means for displaying the electronic medical chart corresponding to the selected diagnosis order information, and when the electronic medical chart is displayed, the diagnosis recording information identification information of the diagnosis recording information included in the electronic medical chart registered being made correspondent to the data base memory means, and by using the correspondence of the diagnosis recording information identification information and the photographic image identification information, a means by which the diagnosis recording information included in the electronic medical chart, and the photographed image data corresponding to the diagnosis recording information are read and displayed, is provided.

The invention written in item 44 is characterized in that: in the photographic image diagnosis system for medical service written in any one of items 40 to 43, the diagnosis system comprises an electronic stethoscope to collect auscultatory sound from a patient and to output auscultatory sound data, the auscultatory sound data outputted from the electronic stethoscope and auscultatory sound identification information to identify the auscultatory sound data are correlated and registered in the data base memory means, and the auscultatory sound identification information and a diagnosis recording information identification information to identify the diagnosis recording information identification corresponding to the collected sounds of the electronic stethoscope are correlated and registered in the data base memory means.

The invention written in item 45 is characterized in that: in the photographic image diagnosis system for medical service written in item 44, the terminal device selects a specific diagnosis order information from the diagnosis order information in response to an operation input and is provide with a means to indicate a electronic medical chart corresponding to the selected diagnosis order information, and is provided with
a photographed image display means to read out and indicate the diagnosis recording information included in the electronic medical chart and a photographed image corresponding to the diagnosis recording information by using the diagnosis recording information identification information of the diagnosis recording information included in the electronic medical chart registered with correlation in the data base memory means and the correlation of the diagnosis recording information identification information and the photographed image identification information when indicating the electronic medical chart, and
an auscultatory sound display means to read out and indicate the diagnosis recording information included in the electronic medical chart and the auscultatory sound data corresponding to the diagnosis recording information by using the diagnosis recording information identification information of the diagnosis recording information included in the electronic medical chart registered with correlation in the data base memory means and the correlation of the diagnosis recording information identification information and the auscultatory sound identification information when indicating the electronic medical chart.

The invention written in item 46 is characterized in that: it is a photographic image diagnosis system for medical service having the terminal device to display the electronic medical chart, and the data base memory means connected to the terminal device; in the data base memory means, the medical chart identification information for identifying the electronic medical chart is made correspondent for each electronic medical chart and registered; further, in the electronic medical chart, the diagnosis recording information is included; and the diagnosis recording information identification information for identifying the diagnosis recording information is made coincident for each diagnosis recording information and registered; the terminal device selects the specific diagnosis order information from the diagnosis order information by the operation input; and a means for displaying the electronic medical chart corresponding to the selected diagnosis order information; and when the electronic medical chart is displayed, by using the correspondence with the diagnosis recording information identification information of the diagnosis recording information included in the electronic medical chart registered being made correspondent to the data base memory means, a means is provided by which the diagnosis recording information included in the electronic medical chart, and the photographed image data corresponding to the diagnosis recording information are read out and displayed.

The invention written in item 47 is characterized in that: in the photographic image diagnosis system for medical service written in item 46, the terminal device is provided with an auscultatory sound display means to read out and indicate the diagnosis recording information included in the electronic medical chart and the auscultatory sound data corresponding to the diagnosis recording information by using the correlation of the diagnosis recording information identification information and the auscultatory sound identification information when indicating the electronic medical chart.

In the photographic image diagnosis system for medical service written in any one of items 43 to 47, the invention written in item 48 is characterized in that: the terminal device is provided with a display means by which the diagnosis recording information included in the electronic medical chart and the photographed image data corresponding to the diagnosis recording information are made correspondent to each other, and displayed.

In the photographic image diagnosis system for medical service written in items 45 or 47, the invention written in item 49 is characterized in that: the terminal device is provided with a display means by which the diagnosis recording information included in the electronic medical chart, the photographed image data corresponding to the diagnosis recording information and the auscultatory sound data corresponding to the diagnosis recording information are made correspondent to each other, and displayed.

In the photographic image diagnosis system for medical service written in any one of items 40 to 49, the invention written in item 50 is characterized in that: the terminal device is corrected to an information system in the hospital, and from the information system in the hospital, the diagnosis order information is received.

According to the invention of item 1, because the present invention is for medical service, the use in the room is a premise, and there is no case where a light amount of the light from the outside is too large, and so, it is difficult that the display of the display section is discriminated. Further, because the light of the displayed photographic related information is guided to the pupils together with the light from the outside, and the photographic related information is presented by being superposed on the image of the outside, while the doctor directly examines the patient, he confirms the photographic related information and can photograph it.

According to the invention of item 2, it is of course that the same effect as the invention written in quoted items can be obtained, because the photographing range information can be confirmed, the doctor can recognize whether the diseased part of the patient is within the photographing range, and the photographic miss can previously be suppressed.

According to the invention of item 3, it is of course that the same effect as the invention written in quoted items can be obtained, and the camera section can change the focal length, and also when the doctor does not move forwards and rearwards, he can photograph the image of the diseased part in an adequate size, and because the photographing range information corresponding to the focal length of the camera section at the time of photographing, is displayed on the display section, while the doctor directly inspects the patient, he can confirm the photographing range corresponding to the focal length of the camera section at the time of photographing. Therefore, the doctor can perceive whether the diseased part of the patient is within the photographing range, and the photographic miss can be previously suppressed.

According to the invention of item 4, it is of course that the same effect as the invention written in the quoted item can be obtained, and when the camera section and display section are positioned at different positions, it is not necessary that the camera section is provided in front of the eye-part of the applied person, and because the patient can look the eyes of the doctor, it is few that the patient have the psychological sense of discomfort such as the uneasiness. However, the parallax is generated thereby, however, because the photographing range information corresponding to the focus distance of the camera section is displayed on the display section, the photographer can photograph while he confirms the photographing range to be photographed, and the photographic miss can be previously prevented.

According to the invention of item 5, it is of course that the same effects as the invention written in quoted items, can be obtained, because the focusing position information is presented to the pupils of the photographer by being superposed on the image of the outside, the doctor can confirm the focusing position and photograph while he directly consulting the patient. Therefore, he can accurately photograph the diseased part of the patent.

According to the invention of item 6, it is of course that the same effect as the invention written in quoted items can be obtained, because the focus position information corresponding to the focusing distance of the camera section is displayed on the display section, while the doctor directly examines the patient, he can confirm the focusing position and conduct the photographing, therefore, the photographic miss can be previously suppressed.

According to the invention of item 7, it is of course that the same effect as the invention written in quoted items can be obtained, because the focus information corresponding to the focusing of the camera section is displayed on the display section, while the doctor directly examines the patient, he can confirm the focusing information and conduct the photographing, therefore, the photographing miss can be previously prevented.

According to the invention of item 8, it is of course that the same effect as the invention written in quoted items can be obtained, because the photographic operation information corresponding to the photographic operation of the camera section is displayed on the display section, while the doctor directly examines the patient, he can recognize the photographic operations. Therefore, the photographic miss can be previously prevented.

According to the invention of item 9, it is of course that the same effect as the invention written in quoted items is obtained, because the photographing start information corresponding to the photographing start instructing information of the camera section is displayed on the display section, the doctor can recognize that the photographing is conducted while he directly inspects the patient. Therefore, the photographic miss is previously suppressed.

According to the invention of item 10, it is of course that the same effect as the invention written in quoted items is obtained, because the photographing ready information corresponding to the photographing ready instructing information of the camera section is displayed on the display section, the doctor can recognize that the photographic readiness is conducted while he directly inspects the patient. Therefore, the photographic miss is previously suppressed.

According to the invention of item 11, it is of course that the same effect as the invention written in quoted items is obtained, because the flash information corresponding to the flash photographing information is displayed on the display section, the doctor can recognize whether the flash photographing is conducted, while he directly inspects the patient. Therefore, the photographic miss by selection miss whether the flash photographing is conducted, is previously suppressed.

According to the invention of item 12, it is of course that the same effect as the invention written in quoted items is obtained, because the specific position information showing the specific position is displayed on the display section, the doctor can recognize the specific position obtained by the camera, while he directly inspects the patient. Therefore, the photographic miss by the false recognition of the specific position is previously suppressed. According to the invention of item 13, it is of course that the same effect as the invention written in quoted items is obtained, because the indicator position information showing the specific indicator position is displayed on the display section, the doctor can recognize the specific indicator position obtained by the camera, while he directly inspects the patient. Therefore, the photographing miss by the false recognition of the specific indicator position is previously suppressed.

According to the invention of item 14, it is of course that the same effect as the invention written in quoted items is obtained, because the indicator position information showing the position of the stethoscope or the indicator position provided corresponding to the stethoscope is displayed on the display section, the doctor can recognize the position relating to the stethoscope obtained by the camera, while he directly inspects the patient. Therefore, the photographic miss by the false recognition of the position relating to the stethoscope or the false acquisition of the stethoscope position is previously suppressed.

According to the invention of item 15, it is of course that the same effect as the invention written in quoted items is obtained, because the indicator position information showing the position of the flash separately provided from the digital camera for medical service, or the indicator position provided corresponding to this flash is displayed on the display section, the doctor can recognize the position relating to the flash obtained by the camera, while he directly inspects the patient. Therefore, the photographic miss by the false recognition of the position relating to the flash or the false acquisition of the position relating to the flash is previously suppressed.

According to the invention of item 16, it is of course that the same effect as the invention written in quoted items is obtained, because the flash illumination direction information showing the flash illumination direction of the photography operating device is displayed on the display section, the doctor can recognize the flash illumination direction obtained by the camera, while he directly inspects the patient. Therefore, the photographic miss by the false recognition of the flash illumination direction or the false acquisition of the flash illumination direction is previously suppressed.

According to the invention of item 17, it is of course that the same effect as the invention written in quoted items can be obtained, because the applied part is a glass-type, there is no sense of discomfort for both of the doctor and patient, and further, because it is easy to put on and to take off, the doctor can devote himself on the diagnosis without any burden.

According to the invention of item 18, it is of course that the same effect as the invention written in quoted items can be obtained, when the camera section is fixed at almost center of the face, the photographing range of the camera section is practically in the range which can be seen by both eyes of the doctor, and an accident in which, although it is seen by the doctor, a picture is not taken in the photographed image, is hardly generated.

According to the invention of item 19, it is of course that the same effect as the invention written in quoted items can be obtained, because a position of the camera section is at the same height as the pupils of the doctor who conducts the photographing, the parallax in the vertical direction can be decreased between the photographing range of the camera section and eyes of the photographer.

According to the invention of item 20, it is of course that the same effect as the invention written in quoted items can be obtained, because the nose pad can be adjusted vertically, when it is adjusted corresponding to the various features of faces, the parallax in the vertical direction can be decreased between the photographing range of the camera section and eyes of the photographer.

According to the invention of item 21, it is of course that the same effect as the invention written in quoted items can be obtained, the image to be photographed by the camera section is displayed on the display section and the doctor who conducts the photographing, can observe it through the optical system. Therefore, because there is no operation by which the doctor looks the viewfinder, he can conduct the photographing without interrupting the diagnosis.

According to the invention of item 22, it is of course that the same effect as the invention written in quoted items can be obtained, because the hologram has a function of a diffractive element, it can conduct the diffraction reflection different from the geometric regular reflection as a mirror. Therefore, the size of the optical system can be reduced, and the reduction of the thickness of the prism can be intended. Further, a compact arrangement structure of optical elements becomes possible.

According to the invention of item 23, it is of course that the same effect as the invention written in quoted items can be obtained, when the reflection type hologram of so-called volume-type having the thickness, and of the phase type in which the light absorption is few, is used, the display image in which the diffraction efficiency is high, and which is bright, and the outside image can be obtained.

According to the invention of item 24, it is of course that the same effect as the invention written in quoted items can be obtained, because the hologram can have the optical power even when it is a plane, it functions as a lens by the diffraction reflection for the light of the display image, on the one hand, for the outside light, it has no power, and the photographer can conduct a natural observation.

According to the invention of item 25, it is of course that the same effect as the invention written in quoted items can be obtained, the reflection angle has a diffractive reflection angle larger than the regular reflection angle by a diffractive reflection of the hologram. That is, when the same angle is made a target, the inclination can be made smaller than in the regular reflection when the hologram surface is used. By this fact, the prism can be made thin.

According to the invention of item 26, it is of course that the same effect as the invention written in quoted items can be obtained, when the reflecting surface is arranged at an angle opened to incident side of the image light on the prism, the image display section can be arranged at almost right above the prism, the entire optical system can be structured thin.

According to the invention of item 27, it is of course that the same effect as the invention written in quoted items can be obtained, a function as a lens is provided on the polarizing correction member, and when the diopter correction function is given to it, it can also be used for the normal glass.

According to the invention of item 28, it is of course that the same effect as the invention written in quoted items can be obtained, when the polarizing correction member and the reflecting surface arranged each other, have respective surfaces on the same plane, the surface of the plane has continuity, and there is no sense of discomfort in appearance, and it can be used in a natural mode.

According to the invention of item 29, it is of course that the same effect as the invention written in quoted items can be obtained, the image light incident from the prism incident side, is diffracted on the hologram surface while it is alternately reflected between reflecting surfaces which forms almost parallel.

According to the invention of item 30, it is of course that the same effect as the invention written in quoted items can be obtained, because the reflection between the reflecting surfaces is a total reflection, a light amount of the image light is not attenuated and reaches the hologram surface. Further, because the outside light is not cut-off, a broad outside observation range can be secured.

According to the invention of item 31, it is of course that the same effect as the invention written in quoted items can be obtained, when the hologram surface is made a plane, the image light is diffracted in the broad range, and the photographer can observe a larger image.

According to the invention of item 32, it is of course that the same effect as the invention written in quoted items can be obtained, when the opposing reflecting surfaces are made curved surfaces, the aberration correction function is given to it, and the improvement of the image quality can be conducted.

According to the invention of item 33, it is of course that the same effect as the invention written in quoted items can be obtained, and the photographing range information can be displayed to the wide photographing, further, the eclipse by the lens barrel portion due to a case where the lens diameter is too large, is suppressed, which is preferable.

According to the invention of item 34, the doctor can conduct the illumination to the diseased part and the photographing start indicative operation by one operation device, and the diagnosis can be effectively advanced.

According to the invention of item 35, it is of course that the same effect as the invention written in quoted items can be obtained, when the photographing ready information is transmitted and after the photographing readiness is conducted on the digital camera side, the photographing start indicative operation is conducted, the photographing start by the false operation is suppressed, the time lag from the photographing start indicative operation to the photographing is reduced, and the faff-feeling of the photographing or the discomfort of the photographic image is suppressed.

According to the invention of item 36, it is of course that the same effect as the invention written in quoted items can be obtained, the doctor can conduct the illumination to the diseased part and the command operation of the focal length, and the diagnosis can be effectively advanced.

According to the invention of item 37, it is of course that the same effect as the invention written in quoted items can be obtained, the doctor can conduct the illumination for the flash photographing and the indicative operation of the photographing start, by one operation device, and the diagnosis can be effectively advanced.

According to the invention of item 38, it is of course that the same effect as the invention written in quoted items can be obtained, because the almost same area as an area which is illuminated to inspect the diseased part is illuminated at the time of the flash photographing, the photographic miss by the false recognition of the illumination area is previously suppressed. Further, the uncomfortable feeling of the photographic image is suppressed.

According to the invention of item 39, it is of course that the same effect as the invention written in quoted items can be obtained, the operability becomes easy. Further, the inspection and the photographing of the cavity of mouth can be easily conducted.

According to the invention of item 40, the terminal device displays the electronic medical chart corresponding to a specific diagnosis order information selected from the diagnosis order information, and in this electronic medical chart, the diagnosis recording information is included and because the diagnosis recording information identification information which identifies this diagnosis recording information, and the photographic image identification information are made correspondent to each other, and registered, a thing is registered in the discriminable manner in which the photographed photographed image data relates to which diagnosis recording information of which electronic medical chart.

According to the invention of item 41, it is of course that the same effect as the invention written in quoted items can be obtained, when a digital camera identification information for identifying the digital camera for medical service is registered in the data base, in the case where a plurality of photographic devices exist, by which photographic device it is photographed, can be specified. Further, when searched by the specific photographic device, the image photographed by the photographic device can also be read.

According to the invention of item 42, it is of course that the same effect as the invention written in quoted items can be obtained, when the date at which the photographed image data is photographed is registered, the photographed date and hour can be specified, further, when searched by a specific date, the image photographed at the date can be read.

According to the invention of item 43, it is of course that the same effect as the invention written in quoted items can be obtained, the terminal device selects the specific diagnosis order information from the diagnosis order information and displays it, further, when the diagnosis recording information of the electronic medical chart is read out, the photographic image corresponding to the diagnosis recording information is read out, and can display it. Thereby, the doctor compares the image photographed in past to the present state of the diseased part, and the judgement of the advance of the disease and state of the recovery can be easily conducted.

According to the invention of item 44, it is of course that the same effect as the invention written in quoted items can be obtained, the terminal device selects the specific diagnosis order information from the diagnosis order information and displays it, and when the diagnosis recording information of the electronic medical chart is read out, the auscultatory sound corresponding to the diagnosis recording information can be read out, displayed or reproduced. Hereby, the doctor compares the auscultatory sound taken in the past time, to the present auscultatory sound, and can easily judge the state of progress and recovery of the disease.

According to the invention of item 45, it is of course that the same effect as the invention written in quoted items can be obtained, the terminal device selects the specific diagnosis order information from the diagnosis order information and displays it, and when the diagnosis recording information of the electronic medical chart is read out, the image and auscultatory sound corresponding to the diagnosis recording information can be read out, displayed or reproduced. Hereby, the doctor compares the image photographed in the past time, and auscultatory sound taken in the past time, to the present image and auscultatory sound, and can easily judge the state of progress and recovery of the disease.

According to the invention of item 46, the terminal device selects the specific diagnosis order information from the diagnosis order information and displays it, further, when the diagnosis recording information included in the electronic medical chart and the photographic image corresponding to the diagnosis recording information are read out and displayed, the doctor compares the image photographed in the past to the present state of the diseased part, and the judgement of the advance of the disease and state of the recovery can be easily conducted.

According to the invention of item 47, the terminal device selects the specific diagnosis order information from the diagnosis order information and displays it, and when the diagnosis recording information included in the electronic medical chart and the auscultatory sound corresponding to the diagnosis recording information are read out, displayed or reproduced, the doctor compares the auscultatory sound taken in the past time, to the present auscultatory sound, and can easily judge the state of progress and recovery of the disease.

According to the invention of item 48, it is of course that the same effect as the invention written in quoted items can be obtained, because the diagnosis recording information included in the electronic medical chart and the photographic image corresponding to this diagnosis recording information are made correspondent to each other, and can be confirmed, the doctor compares the image photographed in the past time to the present state of the diseased part, and the judgement of the advance of the disease and state of the recovery can be easily conducted.

According to the invention of item 49, it is of course that the same effect as the invention written in quoted item can be obtained, because the diagnosis recording information included in the electronic medical chart and the auscultatory sound corresponding to the diagnosis recording information are made correspondent to each other and can be confirmed, the doctor compares the auscultatory sound taken in the past time and the diagnosis result, to the present auscultatory sound, and can easily judge the state of progress and recovery of the disease.

According to the invention of item 50, it is of course that the same effect as the invention written in quoted items can be obtained, and the diagnosis order information can be received from the information system in the hospital.

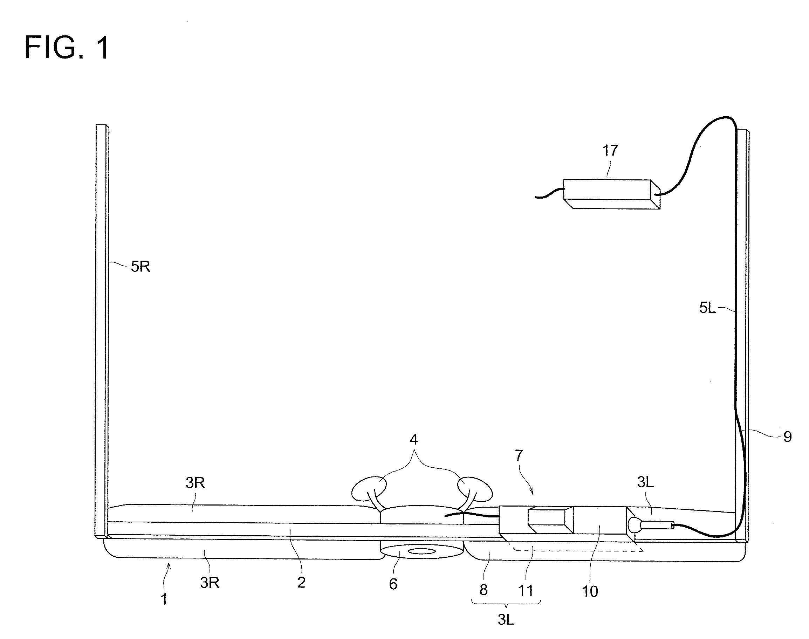

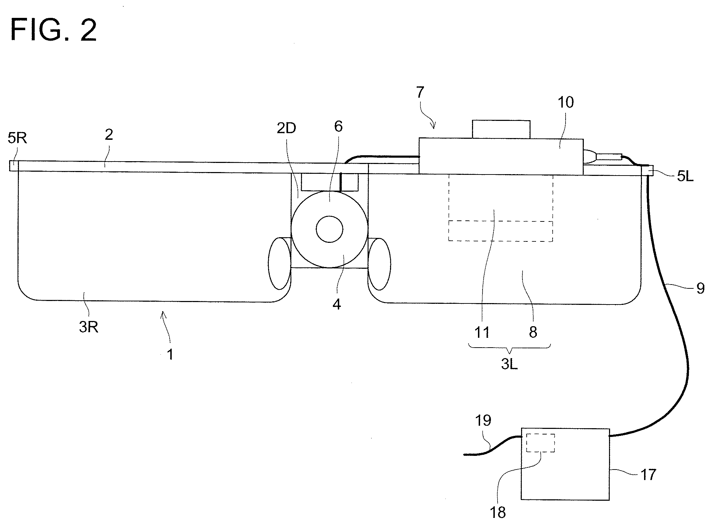

Referring to the drawings, the best mode for carrying out the present invention will be described below. Hereupon, in the description in the present column, there are definitive or assertive expressions, however, they specify the best mode of the invention, and do not limit the extent of the present invention, and do not specify the meaning of terms written in Claims. A general view of a digital camera for medical service 1 in the present embodiment is shown in Fig. 1 (plan view) and Fig. 2 (front view).

The digital camera for medical service 1 has a frame 2, glass lenses 3R, 3L, nose pad 4, temples 5R, 5L, micro camera unit 6, display section 7, cable 9, and control section 17. Then, the display section 7 has a prism 11 and casing 10. Further, the glass lens 3L has a lens section 8 and prism 11. That is, the prism 11 is, as will be described later, a structural component of the display section, and also a structural component of the glass lens 3L. The prism 11 introduces the light of display information together with the outside light to the pupils, and provides the virtual image of this display information by superposing it on the outside image.

The frame 2 supports the upper portion of the glass lens 3R and glass lens 3L. In the vicinity of both ends in the horizontal direction of the frame 2, the temples 5R, 5L respectively extend. Hereupon, in the view, a mode in which the temples 5r, 5L directly connected to the frame 2 through a hinge, is shown, however, the relationship of the frame with the temples 5R, 5L is not limited to this, for example, the temples 5R, 5L may also be attached from the glass lenses 3R, 3L in the vicinity of both ends of the left and right of the applied part through the hinge or not through the hinge. Further, in the frame 2, there is a bridge lower portion 2D, and its both ends support side portions of each glass lens 3R and glass lens 3L.

In one portion of the glass lens for left eye 3L, a prism 11 of the display section 7 is buried, and to the upper portion of the prism 11, a casing 10 for displaying the information from a micro camera unit 6 is attached. To the display section 7 and the micro camera unit 6, a cable 9 for the power supply and the signal sending and receiving is connected. Through this cable 9, the power is supplied from the control section 17, and the digital image data is sent to the control section 17, and sending and receiving of the control signal is conducted to and from the control section 17. Further, the control section 17 is provided with a communication section 18 in its inside, and is connected to a terminal device 20 through the cable 19. Hereupon, cables may also be connected separately for the power supply and for sending and receiving of the photographed image data and control signal. Further, when the distance is short, a structure by which the sending and receiving of the photographed image data and control signal are, by using the infrared communication (IrDA/IrMC) or communication by a electric wave (for example, Bluetooth (R)), conducted by means of wireless, may also be allowed. However, in a case of the cable connection, the communication failure by the positioning of the patient or doctor generated in the wireless communication is not generated, further, there is also practically no bad influence on the cardiopulmonary function auxiliary device buried in the body of patient (as for this, in the case of the electric wave communication, a risk generated when any counter measure is not taken, is high), and this is preferable.

To the bridge lower portion 2D of the frame 2, the nose pad is attached. Further, to the bridge lower portion 2D, the micro camera unit 6 is attached. The nose pad 4 can be position-adjusted vertically. Hereby, both eyes of the photographer and the height of the micro camera unit 6 can be adjusted, and the parallax in the vertical direction can be reduced.

Hereupon, in the present embodiment, an example that it is installed on the photographer by the frame 2 and nose pad 4, is shown, however, it is not limited to this, for example, a mode in which a cap from which the display section 7 and the micro camera unit 6 are suspended, is applied may also be allowable, or a mode in which goggle to which the display section 7 and micro camera unit 6 are fixed, is applied, may also be allowable, and the other mode may also be allowed.

Further, because the micro camera unit 6 is provided in such a manner that it positions at almost the center of the applied part, it positions at almost the center of both eyes, and the subject caught by both eyes of the photographer can be almost photographed with a small eclipse. Hereby, for example, when the swelling of tongue is photographed in the situation in which the patient opens his mouth, it can be photographed with a small eclipse by the lip.

The micro camera unit 6 has an automatic focusing function and a function by which the focal length can be changed. Hereupon, the automatic focusing function is a function by which a focal point is automatically made coincide with the photographic surface of the micro camera. As methods for realizing such an automatic focusing function, there are many methods, however, any one of them may be allowable. Further, as a function by which the focal length can be changed, it is preferable from the diversity of the size of the subject presumed that it is photographed in the diagnosis of the doctor, that the focal length can be changed not less than 3 steps from tele-end to wide-end (particularly, not less than 4 steps), and further, it is preferable that it can be changed not more than 20 steps (particularly, not more than 10 steps), from the view point of the photographic efficiency corresponding to the diagnosis for short time, and the cost reduction.

Then, in the digital camera for medical service 1 , as shown in Fig. 1, the nose pad 4 is brought into contact with the nose, temples 3R, 3L are brought into contact with the ear, side head portion or rear head portion, and it is supported by these 3 points.

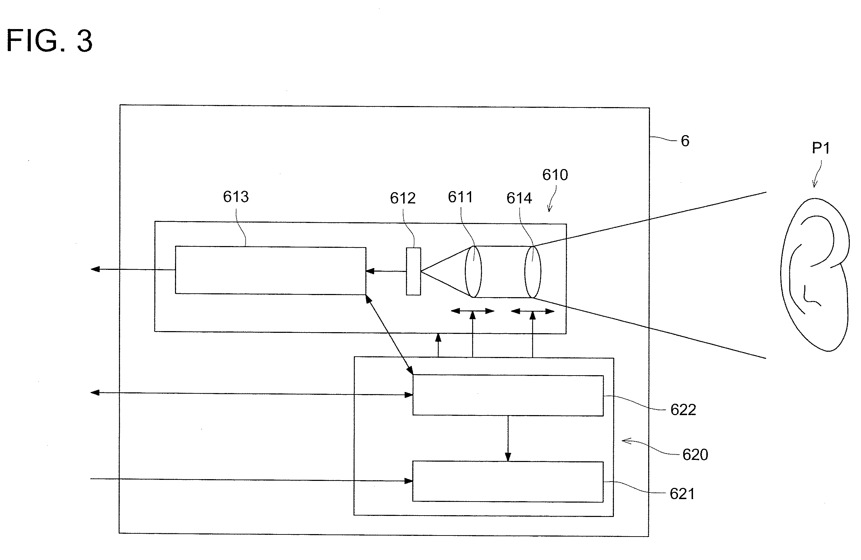

A block structure of the micro camera unit 6 is shown in Fig. 3. In the micro camera unit 6, a camera unit 610 as a mechanism for photographing the subject P, is provided. The camera unit 610 has inside, a focus lens 611 for image-forming the image of the subject P on an image pick-up element 612, and an image pick-up element 612 for converting the subject image image-formed by the focus lens 611 into an electric signal. The image pick-up element 612 can be structured by a CCD (Charge Coupled Device) or a CMOS (Complementary Metal Oxide Semiconductor) or etc. Further, in the camera unit 610, it has a signal processing circuit 613 for processing the image signal converted into the electric signal by the image pick-up element 612, and for outputting the digital image signal, and a zoom lens 614 for changing the photographic magnification of the subject P.

In the imaging by the image pick-up element 612, because the photographing range can be visually confirmed by a visual image as will be described later, it is preferable that the visual image photographing to photograph an image of the photographing wavelength peak within the visual wavelength range (400 - 700 nm) is included. However, not only the visual image photographing, an imaging by which the infrared image (an image whose photographing wavelength peak is more than 700 nm) or ultraviolet image (an image whose photographing wavelength peak is less than 400 nm) can also be photographed, may also be allowable.

Hereupon, in Fig. 3, an example in which it is composed of 2 lens groups and each lens group has respectively one lens, is shown, however, it may include lens groups of 3 groups or more, or lens groups composed of a plurality of lenses. Further, it may also be one lens group.

Then, a drive section 620 attached to the micro camera unit 6 has, in its inside, a motor-driven mechanism 621 which is a drive motive power of the focus lens 611 to conduct the image-formation operation in the camera unit 610, and the zoom lens 614, and a camera control section 622 to control the motive power of the motor-drive mechanism 621. As a drive source of the motor-drive mechanism 621, a small sized motor such as a micro motor or an ultrasonic motor can be used. The camera control section 622 controls the motor-drive mechanism 621 by a command from the control section 17, and controls the drive of the focus lens 611 for focusing and the drive of the zoom lens 614.

Then, the micro camera unit 6 has an automatic focus function, and the signal processing circuit 613 detects an in-focus position on the surface of the image pick-up element 612 when the diseased part P1 of the subject P is photographed on the basis of output of the image pick-up element 612, and the camera control section 622 in which the information relating to the in-focus position is received, controls the motor-drive mechanism 621 to move the focus lens 611, and conducts the focus correction. Then, when the in-focus position is within a predetermined range, the camera control section 622 judges that the image is focused, and transmits in-focus signal and the information of the focus distance at that time to the control section 17.

Further, the micro camera unit 6 has an automatic exposure function having an exposure mode corresponding to the flash photographing and an exposure mode corresponding to the photographing for which the flash is not used. For such an automatic exposure function, a publicly-known automatic exposure method may be used.

Further, by the photographing operation command from the control section 17, the camera control section 622 controls the photographing operation by controlling the signal processing circuit 613 and the motor-drive mechanism 621, and the signal processing circuit 613 receives the image pick-up signal from the image pick-up element 612, and conducts signal processing, and sends the image pick-up image data to the control section 17. Then, the control section 17 transfers the received image pick-up image data to the terminal device 20. Further, the control section 17 controls the display section 7, and the display section 7 displays the photographing operation information corresponding to the photographing operation by such a micro camera unit 6.

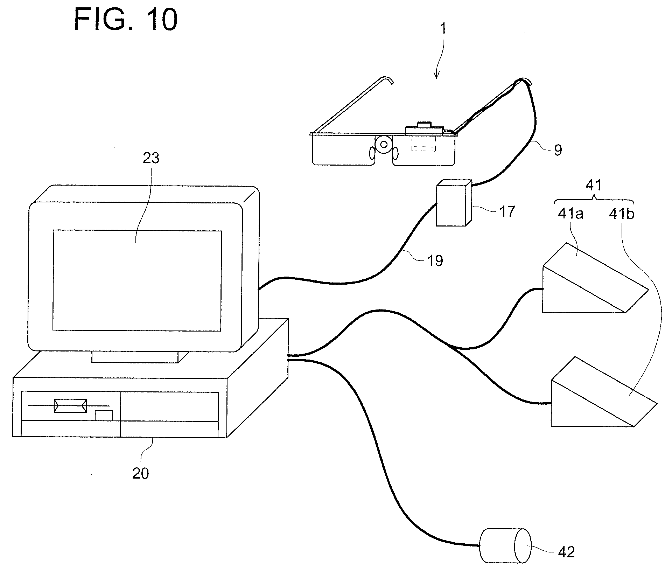

Further, by the zoom command from the terminal device 20 shown in Fig. 10, when the camera control section 622 controls the motor-drive mechanism 621 to move the zoom lens 614, the focal length is changed. Then, the information of the focal length when the photographing operation is conducted by the photographing operation command is transferred from the camera control section 622 to the control section 17. Then, the control section 17 controls the display section 7, as will be described later, corresponding to the received photographic related information, and the display section 7 displays the display information such as a photographic related information.

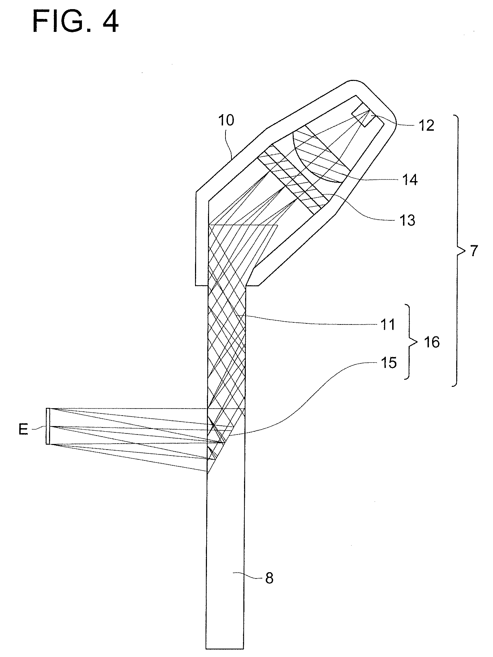

A cross section of the display section 7 is shown in Fig. 4. An upper end portion of the prism 11 is covered by the casing 10. The upper end portion of the prism 11 is sandwiched by the casing 10.

The display section 7 has the casing 10, prism 11, display unit 13, and lens 14. The casing 10 accommodates a display light source 12, display unit 13 and lens 14, and holds them. The display unit 13 is a device for displaying when the transmission of the light from the display light source 12 is controlled, and can be structured by a transmission type LCD or the like. The image information such as the photographing range information from the micro camera unit 6 is displayed. The display light source 12 is a light source for illuminating the display unit 13, and can be structured by an LED (Light Emitting Diode) or a laser diode. In the present example, from the view point of the protection of eyes for the long period of use, the LED is used. The lens 14 is an illumination optical system for uniformly guiding the light emitted from the light source 12 to the front surface of the display unit 13.

The prism 11 is plane, and made of a transparent glass or resins. The prism 11 guides the light of the image displayed on the display unit 13, and presents the virtual image of the image to pupils E of the photographer. The upper end portion of the prism 11 is formed in such a manner that the edge portion is thicker than the inner side, and whose cross section is wedge type, and the casing 10 is attached to the prism 11 in such a manner that it sandwiches the upper end portion whose cross section is wedge-like.

The glass lens 3R is plane, and structured by a single member. Also the glass lens 3L is plane, however, structured by not a single member, but the prism 11 and prism 8. The glass lens 3R and prism 8 are made of the same material as the prism 11, and they does not have the difference of the refractive index. The prism 11 forming the glass lens 3L and the prism 8 have complementary shapes and they are cemented in such a manner that there is no gap, and the surface is continuous. The doctor who installs the digital camera for medical service, observes the outside through the glass lenses 3R, 3L.

The lower end portion of the prism 11 is made in such a manner that, as advancing toward the edge portion, the front surface (surface far from the pupils E) comes close to the rear surface (surface close to the pupils E), and whose cross section is wedge-like. The front surface of a portion whose cross section is wedge-like, that is, the jointed surface with the prism 8 is a plane, and on this plane, a hologram element 15 is formed. The hologram element positions just before the pupils E at the time of installation. The prism 11 and the hologram element 15 structure the ocular optical system 16.

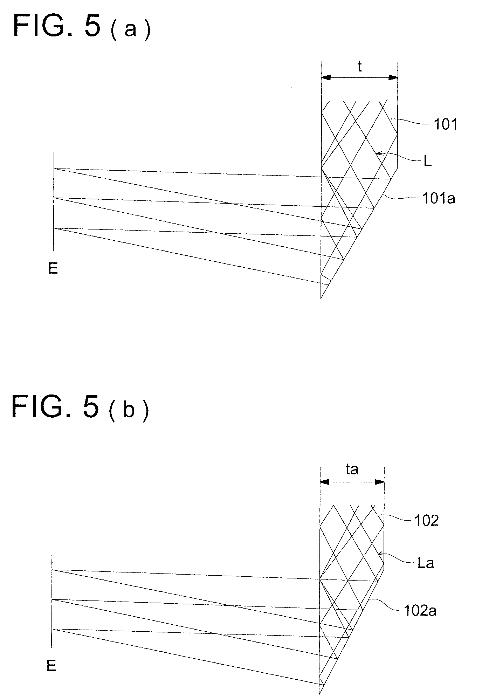

The hologram reflection will be described below. Fig. 5 is a view by which the comparison of the case where a geometric regular reflection is used as the structure by which the display image is guided to the pupils and observed, to the case where the diffractive reflection by the hologram is used, is typically described. Fig. 5(a) shows the case of the geometric regular reflection, and Fig. 5(b) shows the case of the diffractive reflection by the hologram.

In Fig. 5(a), numeral 101 represents a prism which is a light-guide section, numeral 101a represents a concave reflecting surface obliquely arranged on the lower end of the prism 101, and E represents a pupil. Further, in Fig. 5(b), numeral 102 represents a prism which is a light-guide section, numeral 102a represents a hologram surface obliquely arranged on the lower end of the prism 102, and E represents a pupil. Initially, as shown in Fig. 5(a), the light beam L of the display image is transmitted downward in the prism 101, is regularly reflected on the concave reflecting surface 101a, and then is directed to the pupil E while being condensed. On the one hand, as shown in Fig. 5(b), the light beam La of the display image transmitted downward in the prism 102, is diffractively reflected on the hologram surface 102a, and then is directed to the pupil E while being condensed.

Here, as described earlier, a hologram functions as a diffractive element and can achieve diffractive reflection which is different from geometrical regular reflection achieved by a mirror or the like. In other words, regardless of the inclination of a hologram substrate, it is possible to diffractively reflect light in an arbitrary direction, and therefore the size of an optical system is not defined by the geometric conditions.

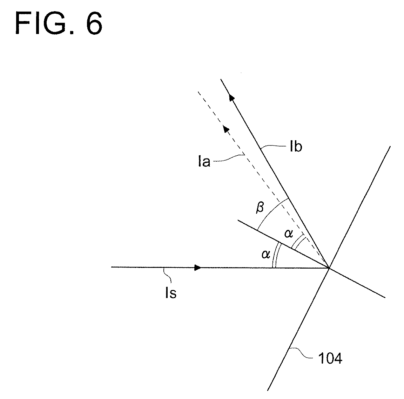

Specifically, for example, as schematically shown in Fig. 6, when incident light 1s having entered a reflecting surface 104 is regularly reflected and the thus obtained reflected light is drawn with a broken line and expressed as 1a, both the incident angle and the reflection angle become .alpha., the same angle. However, when a diffractive reflecting surface is used as the reflecting surface 104, it is possible to make a reflection angle .beta. of a diffractively reflected light 1b that is drawn with a solid line wider than .alpha.. Because of this property, if the direction of the reflected light is the same, compare to the case that the reflecting surface achieves regular reflection, it is possible to make the inclination of the reflecting surface smaller.

Therefore, as shown in Figs. 5A and 5B, respectively, if the position of the pupil E is the same, the hologram surface 102a can be arranged in a less inclined status compare to the concave reflecting surface 101a, and this helps make the thickness ta of the prism 102 thinner than the thickness t of the prism 101a. In addition, although a hologram is formed as a flat surface, it can have optical power, and therefore when the hologram is made to have a see-through function described later, through its diffractive reflection, it functions as a lens element and affects the light beam traveled from the displayed image, on the other hand, it does not affect external light, and this property makes it possible to observe an external image in a natural manner.

On the other hand, when the difference in angles is greater than 90.degree., in other words, in a case of a reflection-type hologram, the range of a diffractive wavelength becomes remarkably narrow compare to a transmission-type hologram, and therefore its wavelength selectivity becomes very high. In other words, a reflection-type hologram has a property that affects a certain wavelength, but does not affect other wavelengths than that. When a reflection-type hologram is used as a combiner for achieving the see-through function which enables an observer to observe a displayed image and an external image simultaneously, because it affects only a certain wavelength, the external light receives little influence from the combiner and this makes it possible to perform see-through observation in a bright and good condition.

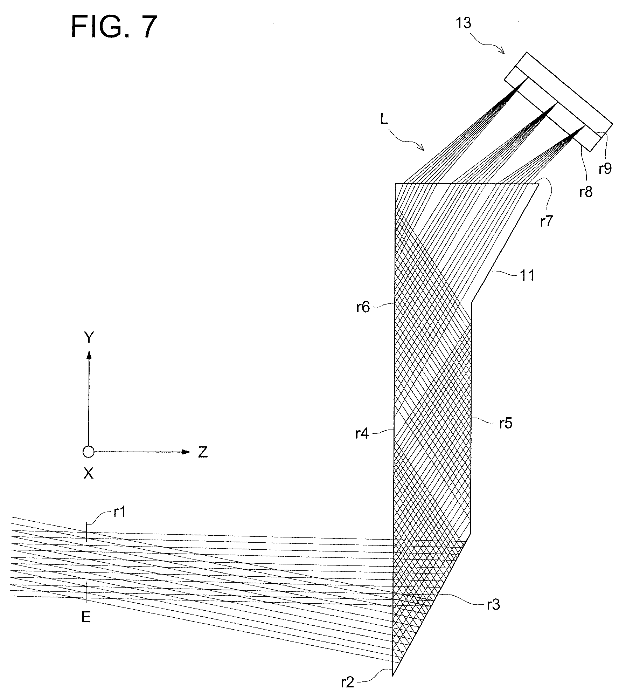

Fig. 7 is a vertical sectional view substantially showing an example of a structure of the optical system in the display section. The prism 11 has a plate-like form obliquely spreading in the upper right direction, and its upper end surface is an incident surface r7. And, on the left and right of the figure, it has the first reflecting surface r6 and the second reflecting surface r5 which face each other with being arranged substantially parallel each other. Further, on a lower end surface, a hologram surface r3 is obliquely arranged in the right direction. On the hologram surface r3, a hologram lens is formed. Further, the first reflecting surface r6 and the hologram surface r3 form a cuneal shape. This first reflecting surface r6 includes light beam selective surfaces r4, r2 which selectively perform total reflection or transmission in accordance with the incident angle, on the same surface.

In the left direction of the figure as seen from the lower end of the prism 11, a pupil E is located. The pupil E has a pupil surface r1. The coordinate system is determined in the following manner. The center of the pupil E is defined as the origin of the coordinate, the forward direction of the pupil E (i.e. rightward of the figure) is defined as the positive of the Z-axis, the upper direction is defined as the positive of the Y-axis, and the plane of the drawing is defined as the YZ-surface. And the direction perpendicularly backward (away from the reader) as seen from the plane of the drawing is defined as the positive of the X-axis. This is true also in the following embodiments. Here, in the upper right direction of the incident surface r7 of the prism 11, an display unit 1 formed of a transmission-type liquid crystal display or the like is arranged, and on its front surface serving as an image display surface r9, an image display member formed of a flat plate glass is arranged. And its front surface is expressed as r8.

As shown in Figure 7, a light beam L conveying a displayed image emitted from the image display surface r9 of the image display element 3 passes through the image display member 4 and exits from its front surface r8, and then enters the incident surface r7 of the prism 11. The light beam L having entered the prism 11 via the incident surface r7 enters the first reflecting surface r6, and then is reflected (total reflection) here. The light beam L reflected from the first reflecting surface r6 enters the second reflecting surface r5 arranged with facing the reflecting surface r6, and then is reflected (total reflection) here. The light beam L reflected from the second reflecting surface r5 enters the light beamelective surface r4, and then is reflected (total reflection) here. The light beam L reflected from the light beamelective surface r4 enters the hologram surface r3.

The wavelength of the light beam L corresponds to the wavelength of the hologram surface r3 in which the diffraction efficiency of the hologram becomes the highest, and the light beam L is reflected on the hologram surface r3. The light beam L reflected on the hologram surface r3 passes through the light beamelective surface r2, and is directed to the pupil surface r1 of the pupil 2. The hologram on the hologram surface r3 has optical power and functions as an eyepiece optical system that enlarges the displayed image for being observed. Because of this property, the light beam L is projected on the observer's pupil while being enlarged. In addition, as shown in Figs. 5(a) and 5(b), in diffractive reflection of a hologram, it is possible to have reflecting angles different from that of geometric regular reflection, and this helps make the inclination of the hologram surface r3 small, and therefore this permits to make the prism 11 thinner.

In this embodiment, a light-guide portion for directing light to the hologram surface r3 of the prism 11 is thinly formed by the construction in which the light beam L is reflected a plurality of times on the reflecting surfaces arranged with facing each other, namely the first reflecting surface r6 and the second reflecting surface r5. In addition, owing to the light beamelective surface for selectively achieving total reflection or transmission in accordance with the incident angles, the light beam L is folded in the optical path, and this makes it possible to output the light beam without separating the optical path, and this helps realize a construction in which each optical component is arranged in a compact manner. Furthermore, the decentered amount of the hologram lens is reduced, and therefore it is possible to obtain a good displayed image with little decentering aberration.

Basically, a hologram exhibits the best wavefront reproducibility and the highest diffraction efficiency when it is given the light beam having the same wavelength and angle as the light beam which formed the hologram. Therefore, it is preferable that the light beam L emitted from the display unit 13 have the strongest luminous intensity at the wavelength in which the hologram lens formed on the hologram surface r3 exhibits the highest diffraction efficiency.

As the digital camera for medical service, as such a peak wavelength, there are many cases where it is in the hue of yellow - red, and from the view point of easiness of discrimination to the diseased part, the wavelength less than 570 nm, particularly, less than 550 nm is preferable, further, from the view point of a stimulus sensitivity of eyes, the wavelength more than 430 nm, particularly, more than 470 nm is preferable. For example, when the hologram having a peak of the diffraction efficiency in the vicinity of 530 nm, is used, and for the display unit 13, a not-self light emission type element such as a liquid crystal is used, as the light source for illuminating this, a green LED having a peak of light emitting strength in the vicinity of 530 nm, is suitable. Because LED has a light emitting wavelength width of a half value width 20 - 40 nm, when this is applied for the light source of the display image light, a structure in which the energy efficiency is good, can be obtained.

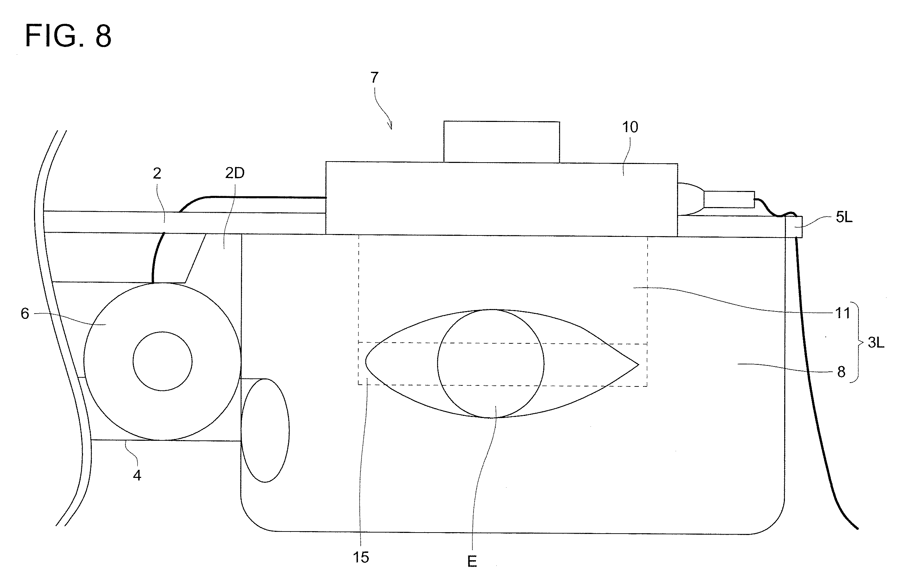

In Fig. 8, a display section 7 of the digital camera for medical service 1 and a position relationship of pupils E of the photographer with the hologram element 15 are shown. It is preferable that the photographing direction of the micro camera unit 6 is adjusted to substantially parallel to the direction of visual field of the pupils of the photographer. Then, along the cross-line of the photographing direction of this micro camera unit 6 with the horizontal plane and the glass surface, the hologram element surface is provided. Further, as shown in Fig. 4 and Fig. 8, it is preferable that the hologram element 15 and the upper end of the prism 11 are substantially parallel. Hereby, because the distance from the upper end of the prism 11 to the hologram element 15 is substantially constant, the production is easy.

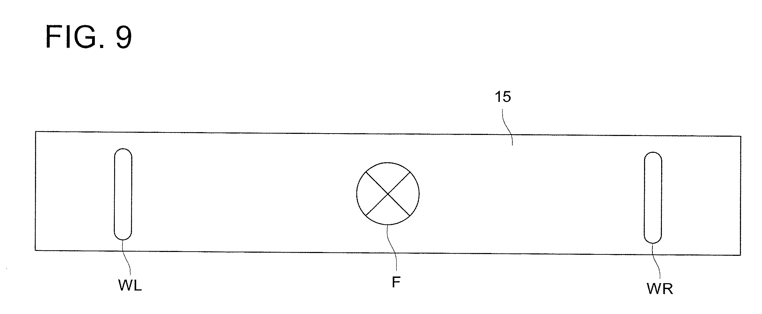

Fig. 9 shows the display area of the hologram element 15 which is viewed from eyes of the photographer. The width of the display area of the hologram element 15, that is, a fact that the width of the display section 7 is larger than the lens diameter of the micro camera unit 6, can display the photographing range information up to the wide angle photographing, further, an eclipse by the lens barrel section when the lens diameter is too large, is suppressed, and which is preferable.

By using Fig. 4 and Fig. 9, the display function will be described below.

The control section 17 controls the display section 7, as will be described below, corresponding to the information such as the photographing ready information, focus signal, focus distance, focal length and the photographing start information, received from the camera control section 622 of the micro camera unit 6, as will be described below, controls the display section 7, and the display section 7 displays the photographing related information such as the photographing ready information, the photographing range information, the focus position, focus information, and photographing start information.

The control section 17 displays the photographing related information such as the photographing ready information, the photographing range information corresponding to the focus distance and focal length, focus position display F at the position corresponding to the focus distance and focal length, and the focus display F corresponding to the focus signal, and the photographing start information, on the display unit 13 of the display section 7. The photographer superposes the virtual image of the photographing related information such as the photographing range information corresponding to the focus distance, the focus position display F and focus display F on the outside image, through the optical system, and can observes them on the surface of the hologram element 15. Herein, the focus position display F and the focus display F are combined with each other, and displayed as a circle, and a part displayed in the circle shows that it is the focus position. Further, at the time of out-of-focus, x-mark is displayed in the circle, and when the x-mark is deleted at the time of in-focus, the photographer can perceive the in-focus.

Herein, the focus position display F is made the circle, however, when it is a method by which the photographer can be perceived, the shape, display color, method do not matter. Further, the focus information F is made to be used both as the focus position display F, however, it is not limited to this. Further, the display at the time of in-focus, out-of-focus, is shown by the existence of x-mark, however, when it is a method by which the photographer can be perceived, which shape, display color, method, may also be allowed.

Further, the control section 17 makes display the frame display WL, WR showing the range in which the micro camera unit 6 photographs, on the display unit 13 of the display section 7. The photographer superposes the virtual image of the frame display WL, WR on the outside image through the optical system, and can observe it on the surface of the hologram element 15.

Hereupon, the smaller the focus distance to the subject is, the larger the parallax between pupils E of the photographer and the micro camera unit 6 is, therefore, the control section 17 controls the frame display WL, WR, displayed by the display unit 13, so that it shows the photographing range in which the influence of the parallax is corrected.

Further, following the transmutation of the photographing range toward the tele(telephoto)-direction and the wide (wide angle)-direction of the micro camera unit 6, the frame display WL, WR corresponding to change of focal length, by the control section 17 provided in the digital camera for medical service, is displayed on the display unit 13 of the display section 7.

When the focal length is short (that is, a case where zooming is conducted to the wide-direction), the photographing range is wide, on the one hand, when the focal length is large, (a case where zooming is conducted to the tele-direction), the photographing range is narrow, therefore, corresponding to that, the control section 17 controls the frame display WL, WR displayed by the display section 13 in such a manner that, when the focal length is small, an interval of the frame display WL, WR is enlarged, and when the focal length is large, an interval of the frame display WL, WR is narrowed, and in such a manner that it shows the photographing range in which the influence of the change of the focal length is corrected.

In this manner, it is conducted in such a manner that the frame display WL, WR near the practical photographing range is displayed. Hereupon, the display of the frame display WL, WR is not limited to a mode described in the present embodiment, and when it is a method by which the photographer can be perceived, which shape, display color, method may also be allowed.

Further, at the time of photographic operation of the digital camera for medical service 1, the photographing completion information showing that the photographing is conducted, is displayed on the display unit 13 of the display section 7, by the control section 17 provided in the digital camera for medical service 1. The photographer superposes the virtual image of the photographing completion information on the outside image, through the optical system, and can observes it on the hologram element 15 surface. In the present example, at the time of photographic operation, when the focus position display-cum-focus display F is flickered, the photographer is perceived. The photographing completion information is not limited to the above display method, and when it is an expression by which the photographer can be perceived, a method does not matter.

Further, in the present embodiment, it is structured such that the information image is offered to left eye of the photographer, however, it may also be structured such that it is offered to right eye or both eyes.

As described above, the display section 7 of the present embodiment is described, however, the display section of the digital camera for medical service of the present invention is not limited to this, but the display section written in Tokkai-2001-305475, the display section written in Tokkaihei 11-64781, or the display section written in Tokkaihei 11-64782, may also be used.

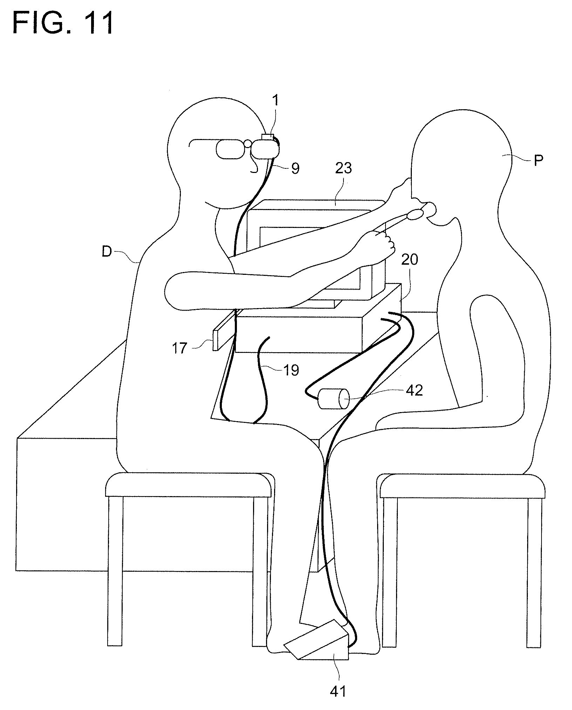

Next, referring to Fig. 10, Fig. 11, Fig. 12, a structure of the digital camera for medial service 1 will be described.

The control section 17 has a CPU (Central Processing Unit), and a memory. The control section 17 controls the micro camera unit 6, and conducts an automatic focusing function, zooming function, or photographic operation. Further, the control section 17 controls the display section 7, and displays the photographic image, focus display F, focus position display F, frame display WL, WR, photographing completion information, on the display section 7. Further, the control section 17 controls the communication section 18 in its inside, and conducts the communication control with the terminal devices 20, 20a.

The communication section 18 is a device for communicating with the terminal device 20. Then, the communication section 18 and the terminal device 20 are connected by a cable 19. As the cable 19, it is preferable that it is a communication line by which the power source can be supplied. Further, it is preferable that the communication section 18 and the cable 19 are based on the serial connection interface standard. As an example of such a communication standard, for example, USB (Universal Serial Bus) or IEEE (Institute of Electrical and Electronic Engineers) 1394 can be listed. Then, the electric power is supplied from the terminal device 20 to the communication section 18, and the control information is sent and received between the terminal device 20 and the communication section 18, and the photographed image data is transferred from the communication section 18 to the terminal device 20.

The terminal device 20 is an information processing device such as a personal computer, and has the display section 23 for displaying the information, operation section 24 for inputting by operations of the operator, communication section 21 for communicating with the digital camera for medical service 1 or a network N, and the control section 22 which calculates based on the information from this, and controls them. The display section 23 is a device for displaying the image or information by the control of the controls section 21. This display section may also be, for example, LCD or CRT. Further, to the communication section 21, a foot switch 41, microphone 42 are connected. The operation section 24 is structured by a key board including numerical key, character key, each kind of function key, for inputting operations, or a touch panel integrally structured with the display section 24, and outputs the operation signal corresponding to the operated key to the control section 24. Hereupon, the operation section 24 may also include other input means such as a mouse. The communication section 21 receives the photographed image data photographed by the digital camera for medical service 1 through the cable 9. The control section 22 displays the photographic image on the display section 23 based on the received photographed image data. Further, the controls section 22 controls the communication section 21, and reads out the information relating to the electric medical chart of the patient P to be consulted by the doctor, through the network N, and controls the display section 23, displays the electronic medical chart on the display section 23. In contrast to that, the doctor D can input the doctor's view, diagnostic result from the operation section 24. Herein, it is defined that the terminal device 20a is the same structure as the terminal device 20.

The foot switch 41 is structured by a zoom operation section 41a and photographic operation section 41b, and the doctor D conducts the operation by one leg, or both legs. The zoom operation section 41a is an operation section for adjusting the focal length of the digital camera for medical service 1. As for a mode of this operation section and a method of operation, various modes and methods can be listed, however, any one of them may also be allowed. Further, by means of them, because both hands of the doctor D are free, the effective operation can be conducted without the diagnosis is interrupted by the photographing.

The microphone 42 is a device for inputting the sound in the diagnosis room to the terminal device 20. In the voice input function of the terminal device 20, previously, the sound and a predetermined operation are made correspond to each other, and the sound collected by the microphone 42 is cooperated with the sound input function provided in the terminal device 20, and the digital camera for medial service 1 is operated. For example, in the sound input function of the terminal device 20, when a pronunciation which is not often pronounced in the conversation of the doctor D (for example, in the case of Japanese doctor, v, f, θ-sound which are not present in Japanese language), and a predetermined operation of the micro camera unit 6 are made correspond to each other, the operation of the micro camera unit 6 can be conducted on the sound input into the microphone 42. Hereby, in the same manner as in the above description, because both hands of the doctor D are not troubled, the doctor D can be intent on the diagnosis of the patient P.

Further, as the photography operating device, an example using a foot switch 42, or an example using the microphone 42, are described, however, instead of them, a penlight type photography operating device 400 shown below, may also be used.

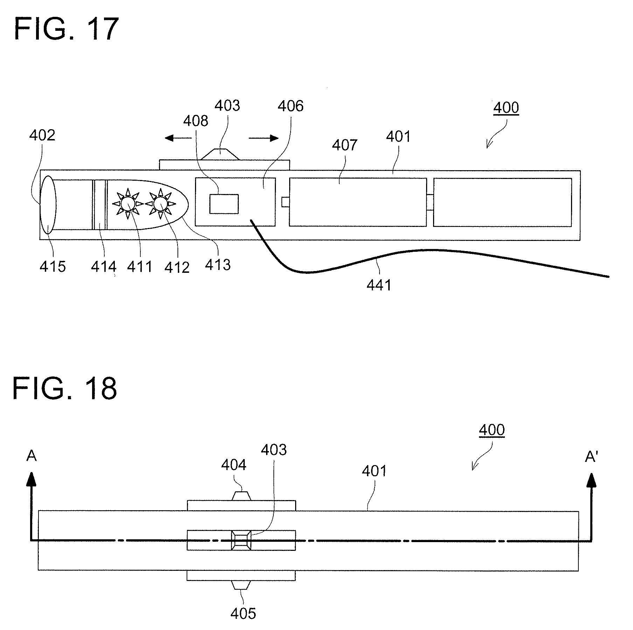

A schematic sectional view of this penlight type photography operating device 400 is shown in Fig. 17, and its schematic upper surface view is shown in Fig. 18. Hereupon, Fig. 17 is a schematic view of A-A' section in Fig. 18. The penlight type photography operating device 400 illuminates from a light projector 402 of a casing 401 end portion, and in its cylindrical casing 401, a battery 407, circuit section 406 driven by the battery 407, light source 411 for the flash photographing which is flashing-light-emitted by the control of this circuit section 406, and illumination light source 412 which is light emitted at a practically constant light amount for conducting an inspection illumination, are housed. Then, these light emissions are projected from the light projection section 402 of the casing 401 end portion. Further, on the surface of the casing 401, operation sections 403, 404, 405 for the operation by the operator are provided, and operations to operation sections 403, 404, 405 are transmitted to the circuit section 406. Further, for the communication between the circuit section 406 and the terminal device 20, a communication line 441 is connected to the circuit section 406, and each kind of information is sent from the circuit section 406 to the terminal device 20 through the communication line 441, or received from the terminal device 20 to the circuit section 406.

Then, in the light which is light emitted in the light source 411 for flash photographing or illumination light source 412, its back light is reflected to the light projection section 402 side in the front side by the curved surface mirror 413, and via a light control section 414 by which the light from the obscured glass is scattered and the light amount distribution is equalized, through a condenser lens 415, it is light projected forward from the projector 402. Hereupon, an example in which the light source for flash photographing 411 and illumination light source 412 are separately provided, is described, however, one light source may also serve both the light source for flash photographing and illumination light source.

Although, in this embodiment, the flash illuminating means and the inspection-useable illuminating means are a common optical system, these may be a separate body.

Operation sections 403, 404, 405 are a slide member which is operated by being slid in the long axis direction of the casing 401. However, it may also be the operation section of other modes such as a press button or touch button. The operation section 403 is a member for being operated by the first finger, and the operation section for inputting that the focal length is lengthened or reduced. The operation section 404 is a member for being operated by the second finger or the third finger, and is the operation section for inputting the selection whether it is the flash photographing or not. The operation section 405 is a member for being operated by the thumb, and the operation section for inputting the photographing ready operation and the photographing start operation. Hereupon, although it is a matter of course, arrangements of operation sections 403, 404, 405, or fingers operating it are not limited to this.

Then, the operation section 405 can indicative-input the photographing start operation after the photographing ready operation is indicative-inputted. Then, the circuit section 406, corresponding to the operation to the operation section 403, transmits the focal length adjusting information to indicate that the focal length is lengthened or shortened, to the terminal device 20. Further, the circuit section 406, corresponding to the operation to the operation section 404, transmits the flash photographing selection information to selectively indicate whether the flash photographing is conducted, to the terminal device 20. Further, the circuit section 406, corresponding to the indicative input of the photographing ready operation to the operation section 405, transmits the photographing ready instructing information to the terminal device 20, and corresponding to the indicative input of the photographing start operation to the operation section 405 after that, transmits the photographing start instructing information. And, the circuit section controls such that the flash photography light source 411 emits light after a predetermined time (not less than 1 seconds and not smaller than 1 seconds) has elapsed after the photography start instruction information is transmitted.

Further, to the circuit section 405, the position detection section 408 is connected, and it detects a space position (a position in 3-dimensional space) and a posture of the photography operating device 400. For the detection of a space position or posture by the position detection section 408, a method by which the acceleration in 3-axis direction and the angular acceleration in the rotation direction are detected by an inertia sensor, and integrated and calculated, or a method by which, from a slippage of timing of the ultrasonic wave received from the ultrasonic oscillator provided at a plurality of positions (4-positions or more are preferable) in the diagnosis room, the position or a posture is detected, is listed, however, it is not limited to this. Then, the circuit 405 transmits the specific position information including the information relating to the space position and posture detected by the position detection section 408 to the terminal device 20. Hereupon, in the present example, the circuit section 405 serves both the control means of the photography operating device and the transmission means, however, it may also be separately structured in such a manner that it is separated into the control circuit and the communication circuit.

Then, the terminal device 20 transmits at once all the information sent from the photography operating device 400 to the communication section 18 of the control section 17 of the digital camera for medical service 1, through its cable 19. The communication section 18 transmits the received information to the control section 17. The control section 17 controls the micro camera unit 6, corresponding to the transmitted information, further, controls the display section 7.

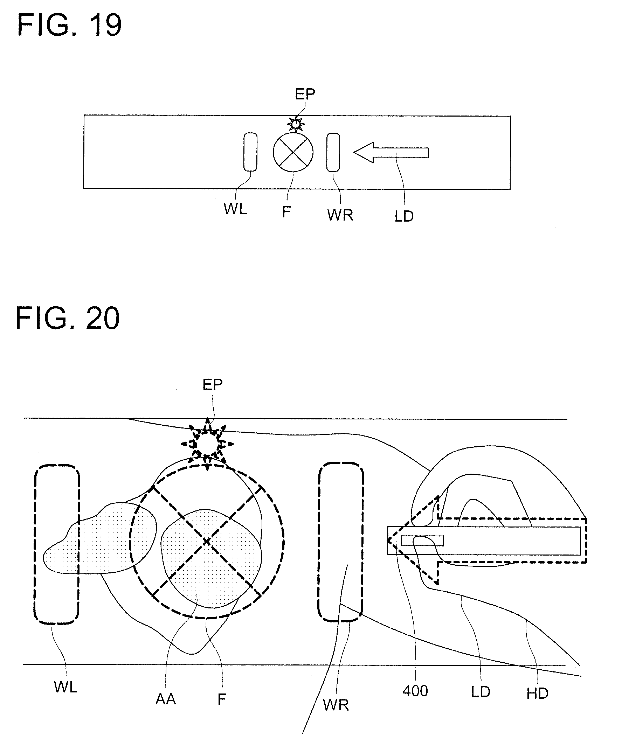

In Fig. 19, an example of the display displayed on the display section 7 when the pen-light type photography operating device 400 is used, is shown. Further, in Fig. 20, an example of the appearance viewed from the photographer is shown.

Then, when the communication section 18 receives the photographing start instructing information from the photography operating device 400, it is transmitted to the control section 17, and the control section 17 outputs the photographing operation command to the camera control section 622 of the micro camera unit 6, based on the received photographing start instructing information, and controls so that the micro camera unit 6 photographs. Further, the control section 17 controls the display section 7 in such a manner that the display section 7 displays the photographing ready instructing information like as the frame displays WL, WR are flickered, corresponding to the reception of the photographing ready instructing information.

Further, when the communication section 18 receives the flash selection information from the photography operating device 400, it is transmitted to the control section 17, and the control section 17 makes the camera control section 622 of the micro camera unit 6 select the optimum exposure mode as the exposure mode of the automatic exposure control, based on the received flash photographing information. Further, when the flash photographing is selected corresponding to the flash photographing information, the control section 17 makes the display section 7 display the flash photographing mark FP.

Further, when the communication section 18 receives the specific position information from the photography operating device 400, it is transmitted to the control section 17, and the control section 17 analyzes it to the display purpose relating to the position and posture of the photography operating device 400 based on the received specific position information, and controls in such a manner that the position information of the photography operating device 400 corresponding to the received specific position information, and an arrow mark LD showing the flash illumination direction are displayed on the display section 7.

In such a manner, it is viewed to the photographer as shown in Fig. 20. In this embodiment, since the illuminating range of the inspection-usable illumination is substantially equal to the illuminating range of the flash illumination, a photographer can estimate an omitted region at the time of a flash photography by confirming directly illumination with the inspection-usable illumination so that the photography operating device 400, that is, the illuminating means can be made a proper position and orientation for a flash photography. In this example, it is an example in which the skin lesions part AA spreading to the right upper portion of the back surface is diagnosis-recorded, and in the case of the skin lesions part, for the confirmation of the presence of curative effect and the progress of prognosis, the comparison to the past image is available.

Hereupon, in the above description, the position detection section 408 for detecting the position and posture is provided on the photography operating device 400 side, however, instead of that, a means for detecting the position of the photography operating device 400 and the flash illumination direction may also be provided on the digital camera for medical service side. As a method to realize this, for example, a method by which the position indicator which is easily recognized by the image recognition from the photographic image is provided in the photography operating device 400, and which controls in such a manner that, according to a position of the position indicator which is photographed periodically on the digital camera for medical service side, and recognized by the image recognition, an arrow mark LD showing the indicator position information of the photography operating device 400 and the flash illumination direction is displayed, is listed, but, it is not limited to this.

Further, the position information and the flash illumination direction are shown by the arrow mark LD, however, it is not limited to such a mode, for example, another mode in which the position information is shown by a circle, and the flash direction is shown by a triangle, may also be allowable. Further, it is preferable that the position information and the flash illumination direction are shown, however, it is also preferable that only the position information is shown, than that it is not shown, further, it is also preferable that only the flash illumination direction is shown, than that it is not shown.

The photography operating device 400 is a pen-light type, and by this, it can be held and operated by a single hand, further, the cavity of mouth can be easily inspected and photographed, further, the skin can be easily illuminated from a plurality of directions and photographed, and the difficulty of the diagnosis from only the recorded image due to the translucent skin can be deleted.

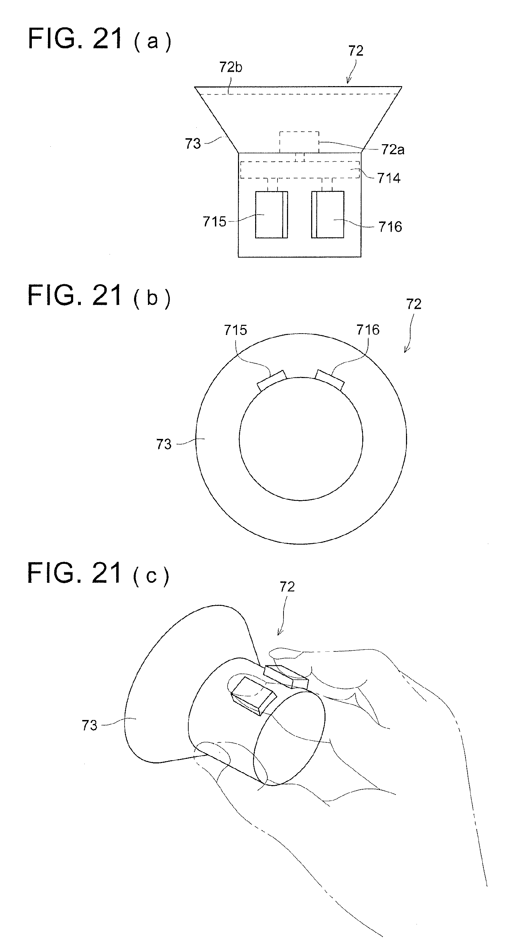

Next, a case where the electronic stethoscope 72 is used, will be described. Fig. 21 is a view showing an electronic stethoscope 72, and Fig. 21(a) is the upper surface view, Fig. 21(b) is a front surface view, Fig. 21(c) is a perspective view showing a state of use. The electronic stethoscope 72 collects an audio sound from the body surface such as a contacted human body and from the obtained electric signal, outputs the auscultatory sound data. As the collection microphone of the electronic stethoscope 72, various microphones can be used, and for example, an electric condenser microphone or a piezo-electric element is used. For example, the electronic stethoscope 72 is provided with, as shown in Fig. 21(a), a condenser microphone 72a, and diaphragm 72b, and a wireless transmission section 714, and switches 715, 716 generating a processing operation indicative signal are provided. Then, as shown in Fig. 21(c), when the first finger is put on the switch 715, and the second finger is put on the switch 716, while the collection microphone 72 is operated by a single hand, the switches 715, 726 can be pressed.

The auscultatory sound collected by the electronic stethoscope 72 and the indicative signal from the switches 715, 716 are transmitted by the electric wave by the wireless transmission section 714 to the terminal device 20. Hereupon, depending on a pattern of a method of pressing of switches 715, 716 or number of pressing times, a indication of a start of collection of the auscultatory sound or end of it, change indication of the collection position or the discard indication of the collected data are transmitted. For example, when the switch 715 is pressed one time, the indication of a start of collection of the auscultatory sound, when the switch 715 is pressed two times in a short time, the re-collection of the auscultatory sound, and the discard indication of the collected data, and when the switch 716 is pressed one time, the change indication of the collection position are transmitted.

The terminal device 20 memorizes each kind of program of the auscultatory sound signal processing system or data, or temporarily memorizes the auscultatory sound data collected by the electronic stethoscope 72 or data data-processed from the auscultatory sound data. Then, not only memorizes the auscultatory sound data transmitted from the electronic stethoscope 72, when the indicative signal of the collection start is received, the terminal device 20 transmits it to the control section 17 of the digital camera for medical service.

Further, by a speaker, not shown, a wide use headphone or a stethoscope type headphone, the auscultatory sound data or the acoustic data data-processed from the auscultatory sound data is acoustic-reproduced. In this case, especially, it may be preferable that a headphone such as a general type headphone or a stethoscope type headphone is linked with a temple 5R, 5L and connected with a control section 17 through a cable and the sound data from the terminal device 20 is reproduced as sound though the control section 17.

Further, the display section 23 of the terminal device 20 displays the auscultatory sound data visualization-processed, characteristic of the auscultatory sound obtained by the data processing, kinds of the disease candidacy or provability corresponding to the disease candidacy.

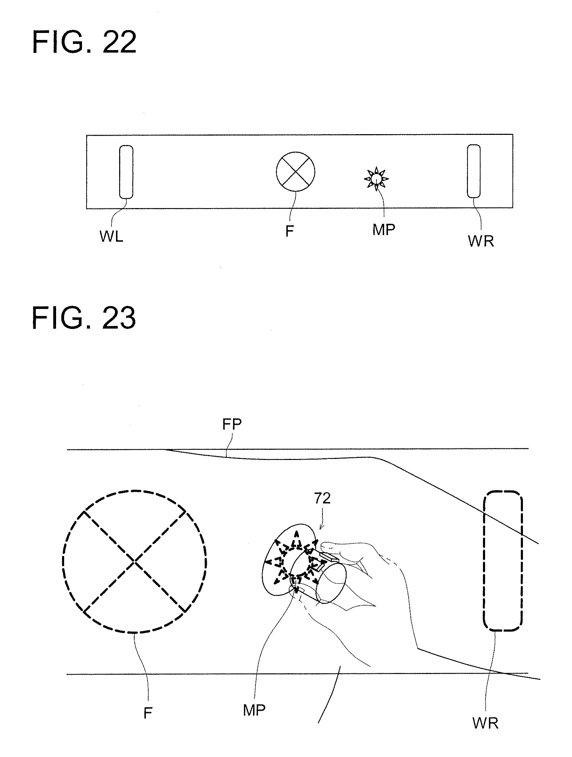

In Fig. 22, an example of the display displayed on the display section 7 when the electronic stethoscope 72 is used, is shown. Further, in Fig. 23, an example of the appearance viewed from the photographer is shown.

Further, in the electronic stethoscope 72, the position indicator 73 which is easily recognized in the image recognition from the photographic image, is provided, and the control section 17 recognizes the position of this position indicator 73 from the image obtained when it is periodically photographed on the digital camera for medical service side by the image recognition. Then, according to the position of the position indicator recognized by the image recognition, the control section 17 controls in such a manner that the display section 7 displays the display MP showing the indicator position information of the electronic stethoscope 72.

Then, when the control section 17 of the digital camera for medical service receives the indication signal of the collection start from the terminal device 20, it photographs the image of an area including the recognized position of the electronic stethoscope 72 at that time, and transmits the information of the position recognized by the electronic stethoscope 72 and the image data of the photographed image to the terminal device 20.

Then, because photographer can look likes as shown in Fig. 23, from this, the photographer can judge whether the position of the electronic stethoscope 72 recognized by the image recognition is correct. Then, when it is recognized at the correct position, by collecting the auscultatory sound, the information of the collecting position of the electronic stethoscope 72 can be automatically obtained.

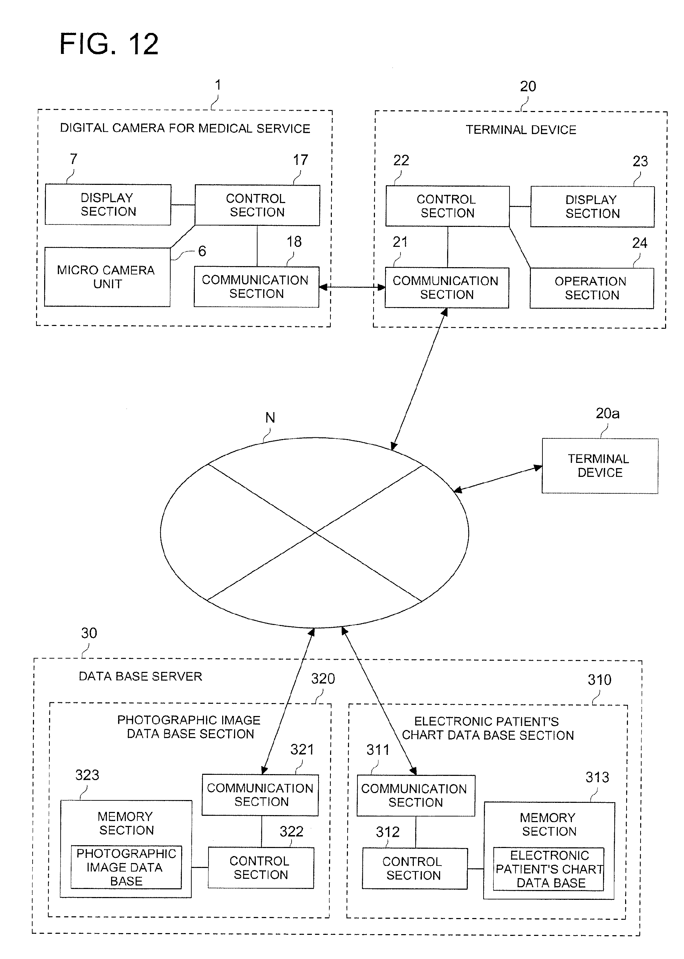

Fig. 12 is a schematic structural view of the photographic image diagnosis system for medical service, using the digital camera for medical service. Hereupon, in the present embodiment, for the digital camera for medical service 1 and the terminal device 20, previously described devices are used. However, the digital camera for medical service in the image diagnosis system for medical service of the present invention is not limited to the previously described digital camera for medical service. As the digital camera for medical service in the image diagnosis system for medical service of the present invention, for example, even when it is a commercial consumer-use digital camera, observation-use or industrial digital camera, further, digital camera written in Tokkaihei 11-341399, further, the digital camera in which design are changed to suit for still photograph from the video camera written in Tokkaihei 11-252419, it may also be allowed. Further, the terminal device of the present invention is not also limited to the previously described terminal device.

Next, a functional structure of the data base server 30 will be described.

The data base server 30 includes an electronic medical chart data base section 310 provided with a communication section 311, control section 312, and memory section 313, and a photographed image data base section 320 provided with a communication section 321, control section 322, and memory section 323.

The communication section 311 is structured by a network interface connected to the network in the hospital N, and a modem, and conducts the sending and receiving of the information to the terminal devices 20, 29a on the network in the hospital N.

The control section 312 is structured by the CPU or the like, and controls across-the-board a processing to change the electronic medical chart data base in the memory section 313, and a processing to search it, by a cooperation of labor with each kind of program stored in its memory.

The memory section 313 is structured by a magnetic memory medium or a semiconductor memory, and has an electronic medical chart data base, and by the control section 312, data change processing such as the renewal, deletion, addition of the data relating to the electronic medical chart, and processing to search the data are conducted.

In the electronic medical chart data base, at least following tables are registered.

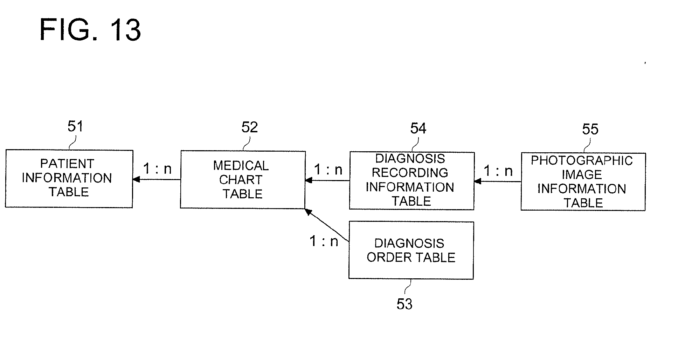

In Fig. 13, the relation view of these 5 tables is shown.

The first table is a table showing the information relating to the patient for each patient ID code. The patient ID code is a unique code for each patient, and the patient identifying information for identifying the patient. As the information relating to the patient, for example, a patient information relating to an attribute of the patient such as a name of the patient, kana name, sex, date of birth, address, and a patient profile information relating to the profile of the patient relating to the diagnosis, are listed. For example, in the present embodiment, as the patient information, a name of the patient, kana name, sex, date of birth, address, are registered. Further, as the patient profile information, allergy, infection, previous disease, living habit, family medial history, are listed. However, as the information relating to the patient, it is not limited to the example of the present embodiment.

The second table is a medical chart table 52 showing a basic information of the medical chart for each medical chart ID code. The medical chart ID code is a medical chart identification information for identifying each one of the medical charts (diagnosis recording). In the medical chart table, other than medical chart ID code, a patient ID code, and for example, the medical chart basic information such as department ID code, first diagnosis date, are recorded. The medical chart ID code is a code proper to each one of medical charts, and the medical chart identification information for identifying the medical charts.

In the medical chart table 52, the relation with the patient information table 51 by the patient ID code, is provided. Then, in the medical chart table 52, record related to one record of the patient information table 51, 0 or 1, more than 1 exist.

As the third table, there is a diagnosis order table 53 showing the information relating to the diagnosis order for each diagnosis order ID code. The diagnosis order ID code is a diagnosis order identification information for identifying each of diagnosis orders. In the diagnosis order table 53, other than the diagnosis order ID code, a medical chart ID code, and the diagnosis order information such as, for example, reception time, the information showing whether the diagnosis is completed (hereinafter, called diagnosis completion information), are registered.

In the diagnosis order table 53, the relation with the medical chart table 52 by the medical chart ID code, is provided. Then, in the diagnosis order table 53 related to one record of the medical chart table 52, 0 or 1 or more than 1 exist.

As the fourth table, there is a diagnosis recording information table showing the diagnosis recording information of the medical chart for each diagnosis recording information ID code. The diagnosis recording information ID code is a diagnosis recording information identification information for identifying each of diagnosis recording information made for each problem in one-time diagnosis. In this diagnosis recording information table, the medical chart ID code for identifying the corresponding medical chart is recorded. As the diagnosis recording information recorded in this diagnosis recording information table, the information such as a diagnosis time, doctor ID code of a doctor in attendance, chief complaint, estimate object, estimate, diagnosis, doctor's view, is recorded.

In the diagnosis recording information table 54, the relation with the medical chart table 52 is provided by the medical chart ID code. Then, in a record of the diagnosis recording table 53 related to one record of the medical chart table 52, 0 or 1 , more than 1 exist.

Further, as a modified example, it may also be structured such that, for one diagnosis recording information, 0 or 1, a plurality of the estimate information can be recorded. In this case, separate from the diagnosis recording information table, an estimate information table showing the estimate information for each estimate information ID code, is provided. The estimate information ID code is an estimate information identification information for identifying each of the estimate information. Then, as the estimate information, the information such as the estimate object, estimate, diagnosis, doctor's view, is recorded. Then, in this case, as the diagnosis recording information, the diagnosis time, doctor ID code of a doctor in attendance, chief compliant, are recorded.

In this case, in a table of the estimate information, the relation with the diagnosis recording information table is provided by the diagnosis recording information ID code. Then, in a record of the estimate information table 53, record related to one record of the diagnosis recording information table 52, 0 or 1, more than 1 exist.

As the fifth table, there is a photographic image information table 55 showing the relationship between the diagnosis recording information ID code and the photographic image ID code. This is a table by which the diagnosis recording information of the patient, and the image data photographed at the time of this diagnosis are made correspondent to each other. The photographic image ID code is a photographic image identification information for identifying each of photographed image data, and when the terminal device 20 receives the photographed image data from the digital camera for medical service 1, it is added as a header part or a file name of the received photographed image data. By this correspondence, when the diagnosis recording information of the electronic medical chart is read, the image photographed at the time of the diagnosis is displayed together with the diagnosis recording information of the patient. Therefore, the doctor compares the past diseased part and condition of a disease to the present one, and judges the progress and recovery condition of the disease, and can conduct an adequate diagnosis.

In the photographic image information table 55, the relation with the diagnosis recording information table 54 is provided by the diagnosis recording information ID code. Then, in the photographic image information table 55, record related to one record of the diagnosis recording information table 54, 0 or 1, more than 1 exist.

Further, in the photographic image information table 55, the information for each photographic image may also be recorded. For example, the camera ID code for specifying the digital camera 1 used for the photographing, the information of the focus distance, exposure information, the information of the focal length, flash photographing information, flash illumination direction or flash position information, which are transmitted from the digital camera 1, marker position information, may also be recorded.

The camera ID code is the information for identifying each camera, hereby, by which camera used in the hospital the recorded image is photographed, can be identified. Then, in this case, although not shown, it is allowable when a camera information table by which the camera ID code and the information relating to the corresponding camera are made correspondent to each other, is recorded. In this camera information table, other than the camera ID code, for example, manufacturer name, name of kinds of devices, device number, supervisor, date of purchase, inspection date, may also be recorded.

Further, when the electronic stethoscope 72 by which the audible sound is collected from the patient and the auscultatory sound data is outputted, is used, and stored in the electronic medical chart, although not shown, as the sixth table, the auscultatory sound information table showing the relationship between the diagnosis recording information ID code and the auscultatory sound ID code is recorded. This is a table by which the diagnosis recording information of the patient and the auscultatory sound data collected at the time of diagnosis of this diagnosis recording are made correspondent to each other. The auscultatory sound ID code is a auscultatory sound identification information for identifying each one of auscultatory sound data, and when the terminal device 20 receives the auscultatory sound data from the electronic stethoscope 721, it is added as the header part or file name of the received auscultatory sound data. By this correspondence, when the diagnosis recording information of the electronic medical chart is read, together with the diagnosis recording information of the patient, the auscultatory sound collected at the time of the diagnosis is image-displayed or reproduced. Therefore, the doctor compares the past and present diseased part and medical condition, and judges the progress of the disease and recovery condition, and can conduct the appropriate diagnosis.

In the auscultatory sound information table, the relation with the diagnosis recording information table 54 is provided by the diagnosis recording information ID code. Then, in the record of the auscultatory sound information table related to one record of the diagnosis recording information table 54, 0 or 1 or more than 1 exist.

Further, in the auscultatory sound information table, the information for each auscultatory sound may also be recorded. For example, a stethoscope ID code for identifying the stethoscope 72 used for collection, the stethoscope position information transmitted from the digital camera for medical service 1 used at the time, or the photographic image ID code of the image data in which this stethoscope 72 is photographed, may also be recorded.

The stethoscope ID code is an information for identifying each of stethoscopes, hereby, by which stethoscope used in the hospital the recorded auscultatory sound is collected, can be specified. Then, in this case, although not shown, it is good when a stethoscope information table by which the stethoscope ID code and the information relating to the corresponding stethoscope are made correspondent to each other, is recorded. In this stethoscope information table, other than the stethoscope ID code, for example, a manufacturer name, name of kinds of devices, number of device, supervisor, purchase date, inspection date, may also be recorded.

Next, the photographed image data base section 320 will be described.

The communication section 321 is structured by a network interface connected to the in-hospital network N, and a modem, and conducts the sending and receiving of the information to the terminal devices 20, 20a on the in-hospital network N.

The control section 322 is comprising a CPU, and controls a processing to data-change the photographed image data base in the memory section 323, a processing to search the data, and a processing to send and receive the data to the terminal devices 20, 20a by a cooperation with each kind of program stored in its memory.

The memory section 323 is structured by a magnetic memory medium or a semiconductor memory, and has a photographed image data base, and in the photographed image data base, the photographed image data in which, to the addition information such as a header information or file name, the photographic image ID code is attached, is stored. Then, the data change processing such as deletion and addition of the data relating to the photographic image, and the data search processing are conducted by the control section 322.

Further, by using the electronic stethoscope 72 by which the auscultatory sound is collected from the patient and the auscultatory sound data is outputted, when it is stored in the electronic medical chart, the auscultatory sound data in which the auscultatory sound ID code is added to the addition information such as the header information or file name, is stored in the photographed image data base. In this case, it may be rather to be called as the acoustic image data base, than called as the photographed image data base, however, for the purpose of simplification of the description, it is called as the photographed image data base. Then, by the control section 322, the data change processing of the deletion and addition of the data relating to the auscultatory sound and the data search processing are conducted.

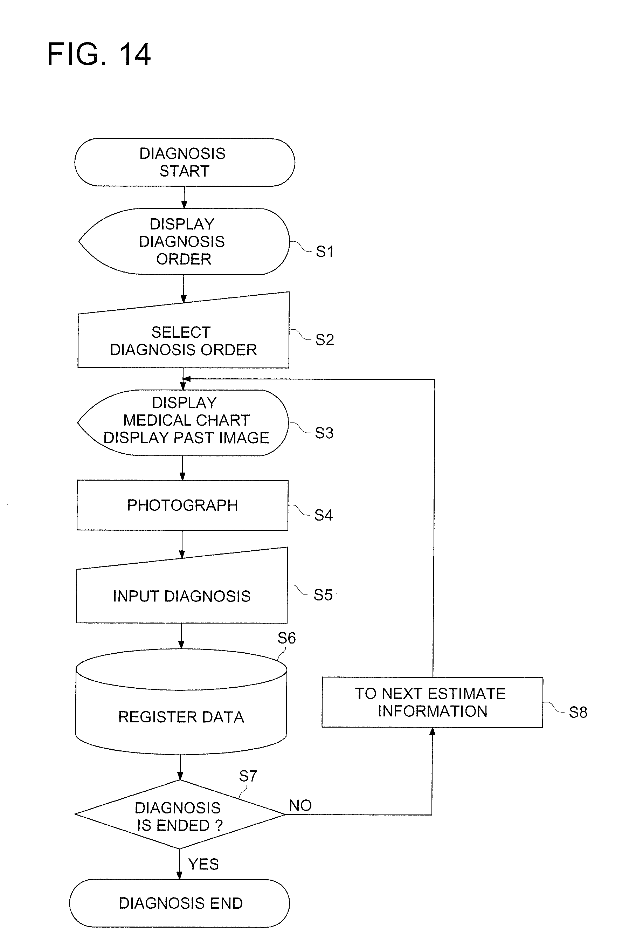

Next, referring to the flow chart in Fig. 14, Fig. 12 and Fig. 15, the content of processing of the photographic image diagnosis system for medical service at the time of diagnosis will be described.

When the doctor examines the patient, he operates the operation section 24 of the terminal device 20. Corresponding to the operation input to the operation section 24, the control section 22 requires the control section 312 through the communication section 21 of the terminal device 20 and the communication section 311 of the electronic medical chart data base section 310, that the diagnosis completion information or diagnosis order information in which the diagnosis is not yet completed, is searched from the electronic medical chart data base. The control section 312, corresponding to the request, searches a record of the diagnosis completion information or un-consulted information, and extracts it. Then, the control section 312 transmits a record of the medical chart table 52 corresponding to the extracted record of the diagnosis order table 53, and a record of the patient information table corresponding to the extracted record of the medical chart table 52 to the terminal device 20. The control section of the terminal device 20, based on the information of received these record, displays the diagnosis order information in which the diagnosis completion information is not yet processed, medical chart information relating to it, and patient information on the display section 23 (step S1).

The doctor operates the operation section 24 so as to select one from the diagnosis order information displayed on the display section 23 by the terminal device 20 (step S2).

The control section 22 sends the diagnosis order ID code of the diagnosis order information selected corresponding to the operation input to the operation section 24, and requires the control section 312 that the read-out of the medical chart basic information, diagnosis recording information and photographic image ID code, (of the sent diagnosis order ID code), corresponding to the diagnosis order information selected by the doctor, is searched from the electronic medical chart data base, through the communication section 21 of the terminal device 20 and the communication section 311 of the electronic medical chart data base section 310. The control section 312, corresponding to the request, based on the diagnosis order ID code sent from the electronic medical chart data base of the memory section 313, extracts the corresponding record from the diagnosis order table 53, and extracts a record of the medical chart table 52 corresponding to the extracted record of the diagnosis order table 53, (for example, by using the patient's ID code of the extracted record of the diagnosis order table 53), a record of the diagnosis recording information table 54 corresponding to the extracted record of the medical chart table 52, (for example, by using the patient's ID code of the extracted record of the diagnosis order table 53), and extracts the photographic image ID code of a record of the photographic image information table corresponding to a record of the extracted diagnosis recording information table 54 (for example, by using the diagnosis recording information ID code of the extracted record of the diagnosis recording information table 53). Then, the control section 32 transmits these extracted records to the control section 22 of the terminal device 20, through the communication section 311 of the electronic medical chart data base section 310 and the communication section 21 of the terminal device 20.

Further, when the auscultatory sound data is stored in the electronic medical chart, the auscultatory sound ID code of the auscultatory sound information table corresponding to the record of the extracted diagnosis recording information table 54 is also extracted further.

Then, the control section 22 requires the control section 322 so that the photographic image information corresponding to the extracted photographic image ID code is read from the photographed image data base, through the communication section 21 of the terminal device 20 and the communication section 321 of the photographed image data base section 320. The control section 322 reads the corresponding photographed image data from the photographed image data base of the memory section 323 corresponding to the request, and transmits the extracted these photographed image data to the control section 22 of the terminal device 20, through the communication section 321 of the photographed image data base section 320 and the communication section 21 of the terminal device 20.

Further, when the auscultatory sound data is stored in the electronic medical chart, further, corresponding auscultatory sound data is also read from the photographed image data base of the memory section 323, in such a manner that the auscultatory sound information corresponding to the extracted auscultatory sound ID code is read from the photographed image data base.

Then, by using the received these records and the photographed image data, control section 22 displays the medical chart basic information, diagnosis recording information, and the photographic image corresponding to diagnosis recording information, on the display section 23 (step S3).

Further, when the auscultatory sound data is stored in the electronic medical chart, further, a button for the indicative input for displaying the auscultatory sound list is displayed on the display section 23.

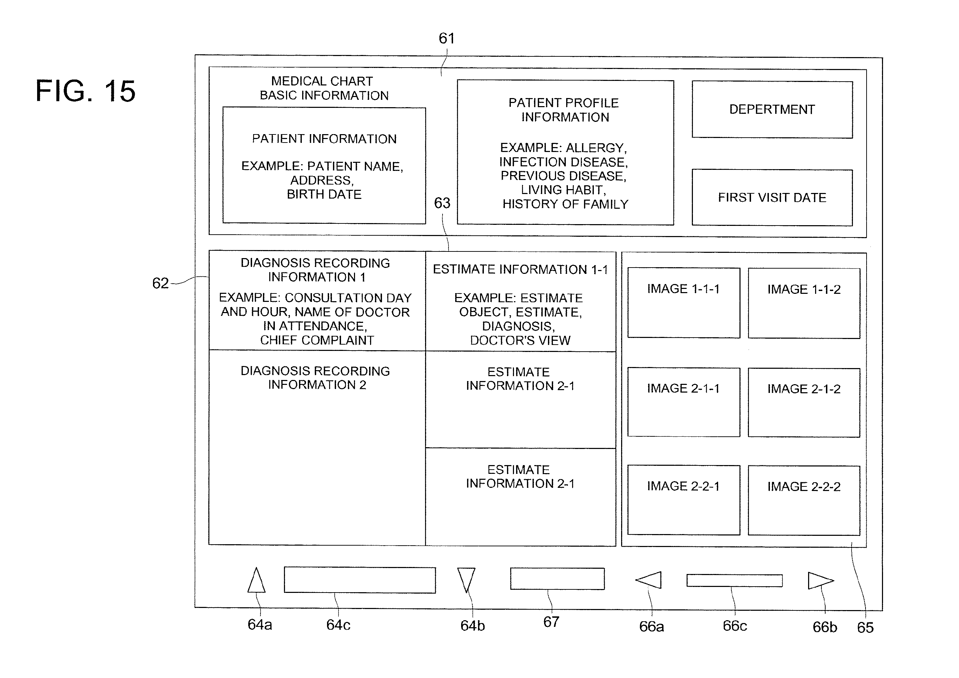

Fig. 15 is an example of the image plane of the medical chart basic information and diagnosis recording information, and photographic image, displayed on the display section 23. The medical chart basic information 61 is displayed in the upper stage of the image plane, and the patient information is enumerated. In the left portion of the middle stage of the image plane, the diagnosis recording information 62 and one or a plurality of estimate information 63 are displayed. The diagnosis history previously conducted on the patient, and this time diagnosis are displayed as a list. Then, by the operation of the upward button 64a and downward button 64b, in the left portion of the lower stage of the image plane, the diagnosis recording information and the estimate information can be scrolled up and down direction. On the estimate information order display frame 64c between the upward button 64a and downward button 64b, the order in the medical chart of the displayed estimate information and the number of all the estimate information in the medical chart are displayed.

On the photographic image frame 65 in the right portion of the middle stage of the image plane, the image photographed at the time of diagnosis, of each diagnosis recording utton information is displayed. By the operation of the leftward button 66a, rightward button 66b on the right side of the lower stage of the image plane, the photographic image related to the diagnosis recording information can be forwarded in order. On the image information display frame 66c between the leftward button 66a and the rightward button 66b, the order of the display image in the photographic images related to the diagnosis recording information, and the number of all images of the photographic images related to the diagnosis recording information are displayed.

When such a display is conducted, the doctor can easily compare the previously photographed image to the condition of present diseased part, and can judge the condition of progress and recovery of the disease of the patient.

Further, in the diagnosis recording information column in which the auscultatory sound data is stored in the electronic medical chart, the button for indicative input for displaying the auscultatory sound list is displayed. Then, when this button is clicked, a list of the auscultatory sound data relating to the diagnosis recording is displayed, and when the specific auscultatory sound data column is clicked, the auscultatory sound of the auscultatory sound data is reproduced, and the analysis data is displayed.

Further, when the doctor photographs the diseased part of the patient by the digital camera for medical service 1, the photographed image data is transmitted to the terminal device 20 (step S4). Further, when the control section 22 receives the photographed image data, it attaches the photographic image ID code which is the unique one in all the system, to the image data. This can be attained when, for example, the serial number is attached to the ID code attached for each terminal device. By controlling the display section 23, the display is switched to the diagnosis recording information and estimate information which are being made up, and by using the photographed image data, the photographic image is displayed on the display section 23, by making it correspond to the diagnosis recording information which is being made up.

Further, in the same manner also when the auscultatory sound data is received, the display is switched to the diagnosis recording information which is being made up, and the estimate information, and the button for indicative input for displaying the auscultatory sound list is displayed. Then, when this button is clicked, the list of the auscultatory sound data relating to the diagnosis recording is displayed, and when the received auscultatory sound data column is clicked, the auscultatory sound of the auscultatory sound data is reproduced, and the analysis data is displayed.

The doctor inputs the result of diagnosis of the present diagnosis to a diagnosis recording column and an estimate information column which are displayed on the display section 23, from the key board, not shown, connected to the operation section 24 (step S5). By the operation input of the operation section 24, the control section 22 controls the display section 23 so that the information inputted to the diagnosis recording column and estimate information column is displayed.

After the operation of step S5, when the data input button 67 of the central portion of the lower stage of the image plane, is pressed, the registration processing of the photographic image which is made correspondent to the diagnosis recording information which is being made up, estimate information, and the diagnosis recording, is conducted. When the control section 22 receives a signal of press of the data input button 67, transmits the medical chart ID code, through the communication section 21 and communication section 311 of the electronic medical chart data base section 310, and requires the data addition of the diagnosis recording information, estimate information and photographic image information, and the data renewal of the diagnosis order information, to the control section 312. The control section 312 adds a new record to the diagnosis table from the received medical chart ID code, diagnosis recording information, estimate information and photographic image information, and by using the diagnosis recording ID code of the added diagnosis recording table, adds a new record to the estimate information table, further, by using the diagnosis recording ID code, adds a new record to the photographic image information table, further, by using the received medical chart ID code, renews the diagnosis completion information of the diagnosis order information to the diagnosis completion.

Further, when the auscultatory sound data is received, further, the control section 22 requires the data addition of the auscultatory sound information to the control section 312. The control section 312 adds a new record of the auscultatory sound information table by using the diagnosis recording ID code.

Further, the control section 22 of the terminal device 20 transmits the photographed image data through the communication section 21 and the communication section 321 of the photographed image data base section 320, and requires the data addition of the photographed image data, to the control section 322. Corresponding to the request, the control section 322 adds and registers the photographed image data into the photographed image data base of the memory section 323 (step S6).

Further, when the auscultatory sound data is received, the control section 22 transmits the auscultatory sound data, through the communication section 21 and the communication section 321, and requires the data addition of the auscultatory sound data to the control section 322. The control section 322, corresponding to the request, adds and registers the auscultatory sound data to the photographed image data base of the memory section 323.

Next, the control section 22 controls the display section 23 and conducts the display requiring the judgement whether the diagnosis is finished. Then, the doctor inputs the judgement whether the diagnosis is finished, by operating the operation section 24. The control section 22, corresponding to the operation input to the operation section 24, judges whether the diagnosis is finished (step S7). When it is judged that diagnosis is not finished, sequence advances to step S8, and a new estimate information is added, and returns again to step 3. When it is judged that the diagnosis is finished, the present processing is ended.

Hereupon, in the present embodiment, the photographic image at the time of diagnosis is made correspondent to the diagnosis recording information of the electronic medical chart, and registered, however, it may also be allowed that, irrespective of the photographed date and hour, the photographic image judged at the time of diagnosis is also made correspondent to the diagnosis recording information and registered. Further, it may also be allowed when the other information obtained at the time of diagnosis, for example, the living body sound information such as the auscultatory sound such as lung sound obtained from a stethoscope or voice sound such as doctor's question and patient answer is, as described above, made correspondent to the diagnosis recording information and registered.

### (Modified example)

Hereupon, an application of the present invention is not limited to the above-described example, but it may be appropriately changed in a scope not departing from the spirit of the present invention.

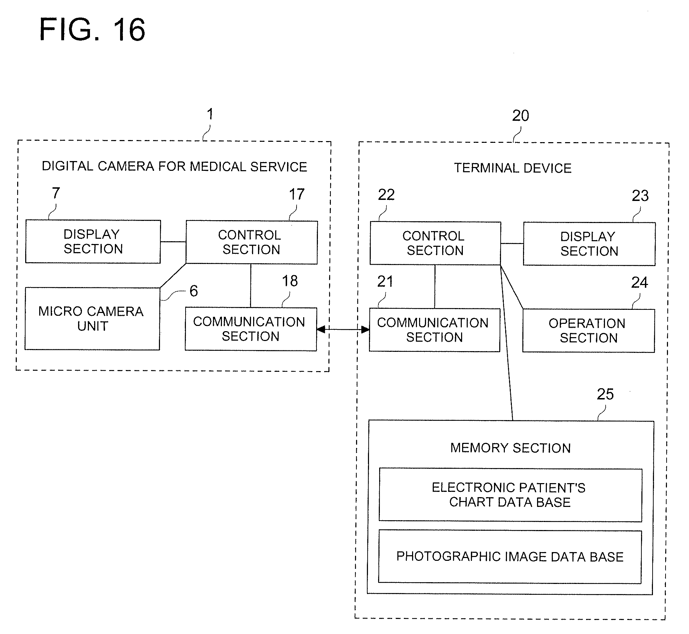

An modified example of the photographic image diagnosis system for medical service will be described below. The present modified example, comparing to the above-described system, as shown in Fig. 16, it is a mode having the electronic medical chart data base and the photographed image data base in the memory section 25 in the terminal device 20, and a point that it is a stand-alone type photographic image diagnosis system for medical service for which the in-hospital network does not exist, is different from the examples described above.

Therefore, a point that, the control section 22 and memory section 25, not though the communication section as in the embodiment 1, renew, delete, add, search the data base inside the terminal device 20, is different from the aforesaid system. Hereupon, the other portions are the same as the above-described system, therefore, the description will be omitted.

## Claims

1. A head-mountable digital camera for medical service, comprising:
a camera section to photograph an object and output digital image of the object;
a display section to form a virtual image of photography relevant information with regard to the photographing by the camera section on a pupil of a photographer through an optical system; and
a mounting member to mount the camera section and the display section on the head of the photographer so as to introduce light of the photography relevant information indicated on the display section to the pupil together with light from the outside so that the virtual image of photography relevant information and the image of the outside are superimposed and formed on the retina of pupil of the photographer.

2. The head-mountable digital camera for medical service described in claim 1, wherein the photography relevant information includes photographing range information to indicate a range photographed by the camera.

3. The head-mountable digital camera for medical service described in claim 2, wherein the camera section is able to change a focal distance and the photographing range information corresponds to a photographing range by the focal distance of the camera section at the time of photographing.

4. The head-mountable digital camera for medical service described in claim 2, wherein the camera section has an automatic focusing function and is positioned in a position different from the display section, and the photographing range information corresponds to a focusing distance of the camera section.

5. The head-mountable digital camera for medical service described in claim 1, wherein the camera section has an automatic focusing function and the photography relevant information includes focusing position information corresponding to a focusing position of the camera section.

6. The head-mountable digital camera for medical service described in claim 5, wherein the camera section is positioned in a position different from the display section and the focusing position information corresponds to the focusing distance of the camera section.

7. The head-mountable digital camera for medical service described in claim 1, wherein the camera section has an automatic focusing function, and the photography relevant information includes focusing information showing that the camera section is in focus.

8. The head-mountable digital camera for medical service described in claim 1, wherein the photography relevant information includes photography operation information corresponding to a photography operation of the camera section.

9. The head-mountable digital camera for medical service described in claim 8, wherein the digital camera comprises a photographing operation receiving section for receiving photography start instruction information from a photography operating device; the camera section conducts photographing based on the photography start instruction information received by the photographing operation receiving section, and the photography relevant information includes photography start information corresponding the receiving of the photography start instruction information.

10. The head-mountable digital camera for medical service described in claim 9, the photography operating device sends photography preparation instruction information before sending the photography start instruction information, the photographing operation receiving section received the photography preparation instruction information from the photography operating device, and the photography relevant information includes photography preparation information corresponding to the receiving of the photography preparation instruction information.

11. The head-mountable digital camera for medical service described in claim 9, the photography operating device sends flash selecting information, the photographing operation receiving section receives the flash photography information from the photography operating device and the photography relevant information includes flash information corresponding to the received flash photography information.

12. The head-mountable digital camera for medical service described in claim 1, further comprising a specific position information obtaining section for obtaining information regarding a specific position with regard to a photography by the camera section, and the photography relevant information includes specific position information indicating the specific position based on the information obtained by the specific position information obtaining section.

13. The head-mountable digital camera for medical service described in claim 12, wherein the specific position is the position of a specific marker, the digital camera comprises a position detecting section for detecting information regarding the position of the specific marker, and the photography relevant information includes marker position information indicating the information regarding the position of the marker detected by the position detecting section.

14. The head-mountable digital camera for medical service described in claim 12, wherein the specific position is a position of a microphone of a stethoscope or the marker provided in correspondence to a microphone.

15. The head-mountable digital camera for medical service described in claim 12, wherein the specific position is a position of a flash of a separated body from the digital camera for medical service or the marker provided in correspondence to the flash.

16. The head-mountable digital camera for medical service described in claim 15, further comprising a flash light irradiating direction obtaining section for obtaining information specifying irradiating direction of the flash, and the photography relevant information includes flash light irradiating direction information based on the information obtained by the flash light irradiating direction obtaining section.

17. The head-mountable digital camera for medical service described in claim 1, wherein the mounting member is a glass-type member including a frame supporting the display section; a nose contact-piece contact with the nose; and an applied part which is attached to near both ends of the frame in the left and right directions, and includes one pair of temples contacting with the ears, side head portion or back head portion.

18. The head-mountable digital camera for medical service described in claim 17, wherein the camera section is provided so as to position in almost center of the applied part.

19. The head-mountable digital camera for medical service described in claim 18, wherein the camera section is provided at a position of almost the same height as the height of pupils of the photographer.

20. The head-mountable digital camera for medical service described in claim 18, wherein the nose-contact piece is adjustable upwardly and downwardly.

21. The head-mountable digital camera for medical service described in claim 1, wherein the display section includes a display unit which displays the information; a casing holding the display unit; and an optical system by which the light from the display unit is guided to the pupils and the virtual image of the image is provided.

22. The head-mountable digital camera for medical service described in claim 21, wherein the optical system comprises a prism having at least two reflecting surfaces arranged in facing each other, and a hologram surface formed of the reflection type hologram; and at least one of the two reflecting surfaces is a light beam selective surface which selectively transmits or reflects light, and the image light from the display unit is reflected between the two reflecting surfaces, next, diffractively reflected on the hologram surface, and after being transmitted through the light beam selective surface, is directed to the pupil of the photographer.

23. The head-mountable digital camera for medical service described in claim 22, wherein the hologram is a volume-hologram and a phase-hologram.

24. The head-mountable digital camera for medical service described in claim 22, wherein the hologram has an optical power by which the image light is enlargedly projected onto the pupils of the photographer.

25. The head-mountable digital camera for medical service described in claim 22, wherein the hologram has a diffractive reflection angle wider than a regular reflection angle observed on the hologram surface.

26. The head-mountable digital camera for medical service described in claim 22, wherein the reflecting surfaces arranged in facing each other have and inclination opening toward the incident side of a prism of the image light beam.

27. The head-mountable digital camera for medical service described in claim 22, wherein the optical system comprises a defelection correction member which corrects deflection of external light transmitting through a prism.

28. The head-mountable digital camera for medical service described in claim 27, wherein the deflecting correction member is attached to the prism, and has surfaces on the same surface of the reflection surfaces arranged in facing each other.

29. The head-mountable digital camera for medical service described in claim 27, wherein the reflecting surfaces arranged in facing each other are substantially parallel to each other.

30. The head-mountable digital camera for medical service described in claim 27, wherein reflection occurring between the reflecting surfaces arranged in facing each other is the total reflection.

31. The head-mountable digital camera for medical service described in claim 22, wherein the hologram surface is plane.

32. The head-mountable digital camera for medical service described in claim 22, wherein at least one of the two reflection surfaces arranged in facing each other is a curved surface.

33. The head-mountable digital camera for medical service described in claim 1, wherein the diameter of a photographing lens of the camera section is smaller than the width of the display section.

34. A photography operating device, comprising:
an operating section for a operator;
an irradiating section for irradiating in response to an operation with the operating section;
a transmitting section to transmit information to a digital camera; and
a control section to control the transmitting section to transmit photography start instructing information in accordance with an operation with the operating section.

35. The photography operating device of claim 34, wherein the control section controls such that the transmitting section transmits photography preparation instructing information in accordance with an operation with the operating section before transmitting the photography start instructing information.

36. The photography operating device of claim 35, wherein the control section controls such that the transmitting section transmits instructing information of a focal distance of the digital camera in accordance with an operation with the operating section.

37. The photography operating device of claim 34, wherein the irradiating section includes a flash light irradiating section for irradiating flash light in accordance with a control by the control section, and the control section controls the flash light irradiating section in correspondence to the control for the transmitting section to transmit the photography start instructing information in accordance with an operation with the operating section.

38. The photography operating device of claim 37, wherein the irradiating section includes a visual diagnosis-usable irradiating section for irradiating for visual diagnosis, and an irradiating direction of the irradiating section is almost equal to that of the visual diagnosis-usable irradiating section.

39. The photography operating device of claim 34, wherein the visual diagnosis-usable irradiating section is a pen-shaped light type.

40. A photographed-image diagnosing system for medial service, comprising:
a digital camera to photograph a patient and to output the photographed image data;
a terminal device connected to the medical-usable digital camera and to display an electronic medical chart;
a data base memory connected to the terminal device,
wherein in the data base memory, medical chart identification information to identify each electronic medical chart is correlated and registered with the identified electronic medical chart, each electronic medical chart contains diagnosis recording information, diagnosis recording information identification information to identify each diagnosis recording information is correlated and registered with the identified diagnosis recording information,
wherein the terminal device selects specific diagnosis order information from diagnosis order information by an operation input and displays the electronic medical chart corresponding to the selected diagnosis order information, and
wherein the photographed image data outputted from the digital camera is correlated and registered with photographed image data identification information to identify the photographed image data in the data base memory, and the photographed image identification information and the diagnosis recording information identifying information to identify the diagnosis recording information corresponding to the photographing by the medical-usable digital camera are correlated with each other and registered in the data base memory.

41. The photographed-image diagnosing system for medial service described in claim 40, wherein the terminal device registers the digital camera identification information to identify the digital camera corresponding to the photographed image identification information in the data base memory.

42. The photographed-image diagnosing system for medial service described in claim 40, wherein the terminal device registers the photographing time information showing time at which the digital camera photographs corresponding to the photographed image identification information in the data base memory.

43. The photographed-image diagnosing system for medial service described in claim 40, wherein the terminal device selects the specific diagnosis order information from the diagnosis order information by the operation input and has a display section to display the electronic medical chart corresponding to the selected diagnosis order information, and when displaying the electronic medical chart, the terminal device reads and display the diagnosis recording information included in the electronic medical chart and the photographed image data corresponding to the diagnosis recording information by using the diagnosis recording information identification information of the diagnosis recording information included in the electronic medical chart registered with the correlation in the data base memory and the correlation of the diagnosis recording information identification information and the photographed image identification information.

44. The photographed-image diagnosing system for medial service described in claim 40, further comprising:
an electronic stethoscope to collect auscultatory sound from a patient and to output auscultatory sound data, wherein the auscultatory sound data outputted from the electronic stethoscope and auscultatory sound identification information to identify the auscultatory sound data are correlated and registered in the data base memory, and the auscultatory sound identification information and a diagnosis recording information identification information to identify the diagnosis recording information identification corresponding to the collected sounds of the electronic stethoscope are correlated and registered in the data base memory.

45. The photographed-image diagnosing system for medial service described in claim 44, wherein the terminal device selects a specific diagnosis order information from the diagnosis'order information in response to an operation input and comprises a display section to indicate a electronic medical chart corresponding to the selected diagnosis order information, and is provided with
a photographed image display section to read out and indicate the diagnosis recording information included in the electronic medical chart and a photographed image corresponding to the diagnosis recording information by using the diagnosis recording information identification information of the diagnosis recording information included in the electronic medical chart registered with correlation in the data base memory and the correlation of the diagnosis recording information identification information and the photographed image identification information when indicating the electronic medical chart, and
an auscultatory sound display section to read out and indicate the diagnosis recording information included in the electronic medical chart and the auscultatory sound data corresponding to the diagnosis recording information by using the diagnosis recording information identification information of the diagnosis recording information included in the electronic medical chart registered with correlation in the data base memory and the correlation of the diagnosis recording information identification information and the auscultatory sound identification information when indicating the electronic medical chart.

46. A photographed image diagnosing system for medical service, comprising:
a terminal device to display an electronic medical chart, and
wherein in the data base memory, medical chart identification information to identify each electronic medical chart is correlated and registered with the identified electronic medical chart, each electronic medical chart contains diagnosis recording information, diagnosis recording information identification information to identify each diagnosis recording information is correlated and registered with the identified diagnosis recording information,
wherein the terminal device selects specific diagnosis order information from diagnosis order information by an operation input and comprises a display to display the electronic medical chart corresponding to the selected diagnosis order information, and
wherein when displaying the electronic medical chart, the terminal device reads and display the diagnosis recording information included in the electronic medical chart and the photographed image data corresponding to the diagnosis recording information by using the diagnosis recording information identification information of the diagnosis recording information included in the electronic medical chart registered with the correlation in the data base memory and the correlation of the diagnosis recording information identification information and the photographed image identification information.

47. The photographed-image diagnosing system for medial service described in claim 46, wherein the terminal device comprises an auscultatory sound display section to read out and indicate the diagnosis recording information included in the electronic medical chart and the auscultatory sound data corresponding to the diagnosis recording information by using the correlation of the diagnosis recording information identification information and the auscultatory sound identification information when indicating the electronic medical chart.

48. The photographed-image diagnosing system for medial service described in claim 46, wherein the terminal device comprises a display device by which the diagnosis recording information included in the electronic medical chart and the photographic image data corresponding to the diagnosis recording information are made correspondent to each other and displayed.

49. The photographed-image diagnosing system for medial service described in claim 46, wherein the terminal device is provided with a display section by which the diagnosis recording information included in the electronic medical chart, the photographic image data corresponding to the diagnosis recording information and the auscultatory sound data corresponding to the diagnosis recording information are made correspondent to each other and displayed.

50. The photographed-image diagnosing system for medial service described in claim 40, wherein the terminal device is corrected to an information system in the hospital, and from the information system in the hospital, the diagnosis order information is received.
